# EUROPÄISCHE PATENTANMELDUNG

(11) **EP 1 707 575 A1**
(43) Veröffentlichungstag der Anmeldung: **04.10.2006**
(21) Anmeldenummer: 05007140.6
(22) Anmeldetag: 01.04.2005
(51) Int. Cl.: C07K 14/47, G01N 33/68

(54) **Ligation synthetischer Zinkfinger-Peptide zu seriellen Bindeproteinen für die spezifische Adressierung doppelstrangiger DNA-Bereiche (Zinkfinger-Sonden)**

(71) Anmelder: Fraunhofer-Gesellschaft zur Förderung der angewandten Forschung e.V., 80686 München (DE)
(72) Erfinder: Birringer, Marc, Dr., 99423 Weimar (DE); von Nickisch-Rosenegk, Markus, Dr., 14979 Grossbeeren (DE); Bier, Frank, Prof.Dr., 14482 Potsdam (DE)
(74) Vertreter: Vossius & Partner

(57) **Zusammenfassung**

Die vorliegende Erfindung beschreibt ein Verfahren zur Herstellung serieller Zinkfinger-Proteine durch die Ligation aus einzelnen artifiziell konstruierten und synthetisch erzeugten Zinkfinger-Peptiden.

Diese Zinkfinger-Proteine sind in der Lage, spezifisch an doppelsträngige DNA-Abschnitte mit entsprechender Länge zu binden und können mittels zusätzlicher Funktionalisierung (z.B. Fluorophore, Biotin) beispielsweise als Sonden an doppelsträngiger DNA (dsDNA), in der flüssigen Phase oder an Oberflächen gebunden, eingesetzt werden.

## Beschreibung

*Die vorliegende Erfindung beschreibt ein Verfahren zur Herstellung serieller Zinkfinger-Proteine durch die Ligation aus einzelnen artifiziell konstruierten und synthetisch erzeugten Zinkfinger-Peptiden. Diese Zinkfinger-Proteine sind* in *der Lage spezifisch* an *doppelsträngige DNA-Abschnitte mit entsprechender Länge zu binden* und *können mittels zusätzlicher Funktionalisierung (z. B. Fluorophore, Biotin) beispielsweise als Sonden an doppelsträngiger DNA (dsDNA), in der flüssiger Phase oder an Oberflächen gebunden, eingesetzt werden.*

Sowohl in der klassischen Molekularbiologie, als auch in der modernen Nanobiotechnologie nehmen die Arbeiten über genetische Erbinformation in Form von Nukleinsäuren, insbesondere mit DNA, einen großen Raum ein. Um bestimmte Bereiche der DNA (Bsp. Gene) zu charakterisieren oder zu detektieren, müssen diese häufig adressiert werden. Dies geschieht in der Regel mit Nukleinsäure-Sonden (Oligonukleotiden), die spezifisch an den entsprechenden Bereichen der DNA hybridisieren und diese so, zumeist mittels fluoreszierenden Farbstoffen, die als Modifikation an den Sonden gebunden sind, kennzeichnen.

Etablierte molekularbiologiche Techniken wie z. B. der "Southern Blot", bei dem doppelsträngige DNA-Fragmente für die Charakterisierung auf Nylonmembranen zu Einzelsträngen denaturiert werden und diese dann, zum Zweck der Hybridisierung mit spezfischen Sonden, auf der Membran verbacken werden, sind in die Nanobiotechnologie nicht 1 : 1 übertragbar. Auf Biochips immobilisierte DNA muss häufig auf ihrer gesamten Länge zugänglich sein, um beispielsweise Enzyme daran arbeiten zu lassen, deren Wirkungsweise in-vitro beobachtet werden soll. DNA-Einzelstränge, die, wie beim "Southern Blot", über ihr gesamtes Phosphat-Rückrat immobilisiert sind, wären hierfür nutzlos, aber für eine eventuelle Adressierung unbedingt notwendig. Zudem ist es sehr aufwendig Einzelstränge zu generieren, wie sie für Experimente an Oberflächen verwendbar sind. Synthetisierte PCR-Fragmente müssen hierzu z. B. über einen aufwendigen enzymatischen Verdau einzelsträngig gemacht werden, bzw. man führt eine nicht immer anwendbare "asymmetrische PCR" durch, welche die gewünschten Einzelstränge generiert.

Die Problematik um das Bereitstellen von DNA-Einzelsträngen zur Hybridisierung mit Oligonukleotid-Sonden bzw. Adressierung an distinkten Orten auf der DNA, erzeugt den Bedarf oder den Wunsch DNA-Doppelstränge zu adressieren.

In jüngster Zeit finden hierbei sogenannte PNA-Sonden Verwendung. PNA ist ein chemisch synthetisiertes einzelsträngiges Nukleinsäure-Analogon mit einem vollständigen Peptid-Rückrat, das lediglich die sterisch richtig orientierten Basen trägt und so mit DNA hybridisieren kann. Der chemische Unterschied macht PNA unempfindlich gegenüber allen DNA-verändernden biologisch-chemischen Agenzien und durch die deutlich höheren Bindungsenergien ist PNA in der Lage in den DNA-Doppelstrang zu hybridisieren und bietet somit die Möglichkeit bestimmte doppelsträngige DNA zu adressieren. Allerdings ist man in der Auswahl der zu hybridisierenden Sequenzabschnitte eingeschränkt, da bestimmte Anforderungen (Länge, Basenabfolge, Basenart) bzw. Einschränkungen an die DNA bestehen. Somit ist PNA auch nicht die Ideallösung. Ein Doppelstrang-DNA-Binder, der uneingeschränkt an frei wählbaren DNA-Abschnitten binden kann war die Motivation, die dem anschließend beschriebenen Verfahren mit Zinkfingerpeptiden als DNA-bindende Moleküle zugrunde lag.

Zinkfinger sind natürlich vorkommende Peptide bzw. Proteine, die beispielsweise spezifisch Promotor-Sequenzen an der DNA binden können und die Transkription beeinflussen (Transkriptionsfaktoren). Naturgemäß findet diese Bindung an doppelsträngiger DNA statt. An den unterschiedlichen Promotoren mit unterschiedlichen DNA-Sequenzen binden spezifische Zinkfinger mit unterschiedlichen Aminosäuresequenzen in der die DNA-Sequenz erkennenden Bindedomäne. Die meist verbreitetsten Zinkfinger haben eine überschaubare Struktur und sind vom sogenannten C2H2-Typ (Abb.: 1), d. h. die für die Struktur verantwortlichen Aminosäuren sind zwei Cysteine (C2)und zwei Histidine (H2) die zentral ein Zinkion binden und so die typische Form dieses Zinkfingers ausbilden. Es sind in der Literatur diverse C2H2-Varianten beschrieben, die unterschiedliche DNA-Motive erkennen. Ein Zinkfinger dieser Art erkennt immer ein Basentriplett.

Die Basen-erkennenden Aminosäuren (-1, 1, 2, 3, 4/L, 5, 6) befinden sich im alphahelikalen Teil des Moleküls (s. Abb. 1) und sind in ihren Positionen genau bestimmt worden (Bsp.: Lu D, Searles MA, Klug A. Crystal structure of a zinc-finger-RNA complex reveals two modes of molecular recognition. Nature. 2003 Nov 6;426(6962):96-100*)*

Durch umfangreiche Literaturrecherche konnte jedem Nukleotid eines Basentripletts (s. o.) die entsprechende korrespondierende Aminosäure an der jeweiligen Position in der alpha-Helix zugeordnet werden. Allerdings sind nicht alle (4³ = 64) Triplettkombinationen in der Literatur beschrieben (Kernliteratur: Nagaoka M, Sugiura Y. Artificial zinc finger peptides: creation, DNA recognition, and gene regulation J. Inorg. Biochem. 2000. 82(1-4):57-63*.;* urt JA, Thibodeau SA, Hirsh AS, Pabo CO, Joung JK. Highly specific zinc finger proteins obtained by directed domain shuffling and cell-based selection. Proc Natl Acad Sci U S A. 2003 Oct 14;100(21):12271-6*;* Choo Y, Klug A. Toward a code for the interactions of zinc fingers with DNA: selection of randomized fingers displayed on phage. Proc Nat1 Acad Sci U S A. 1994 Nov 8;91(23):11163-7*;* u H, Yang WP, Barbas CF 3rd. Building zinc fingers by selection: toward a therapeutic application. Proc Natl Acad Sci U S A. 1995 Jan 17;92(2):344-8*;* Bae KH, Kwon YD, Shin HC, Hwang MS, Ryu EH, Park KS, Yang HY, Lee DK, Lee Y, Park J, Kwon HS, Kim HW, Yeh BI, Lee HW, Sohn SH, Yoon J, Seol W, Kim JS. Human zinc fingers as building blocks in the construction of artificial transcription factors. Nat Biotechnol. 2003 Mar;21(3):275-80*),* so dass wir für jede mögliche Kombination die Triplettmotive extrapoliert haben und auf der Basis eines natürlichen Zinkfingermotivs (SP1 transcription factor) alle theoretischen Bindedomänen konstruiert und eine komplette Datenbank für C2H2-Zinkfingermotive vom SP1-Typ erstellt haben (siehe Anhang).

ABBILDUNG 1:

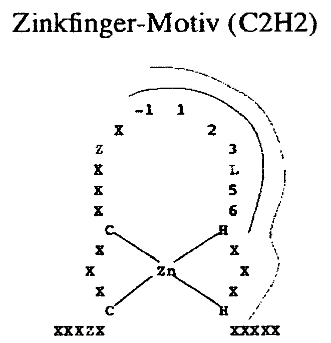

Amino-X-Z-X-C-X₍₂₋₄₎-C-X₍₃₎-Z-X₍₅₎- L-X₍₂₎-H-X₍₃₋₅₎-H-Carboxy

X=Aminosilurc
Z-hydraphobe Amin
L-Luckin
C = Cystein
H= Histid in
-1.1.2.3.5.6 = bindungsrelevante Positionen in der a-fleth

Das natürliche SP1-zinkfinger-Protein besteht aus drei seriell hintereinander geschalteten C2H2-Zinkfingern, die jeweils über einen konservierten Spacer/Linker (Abstandshalter, TGEKP) miteinander verbunden sind. Dieses Protein dient hier als Modell für die hier beschriebenen chemisch synthetisierten Zinkfingermoleküle, die aus mehreren zinkfingerpeptiden beispielsweise bis zu sieben C2H2-Motiven bestehen. Ein solches 7mer würde spezifisch auf einer Nukleinsäuresequenz von 21 Basenpaaren binden.

Im Gegensatz zu schon erteilten Patenten, die sich in vielfältiger Weise mit Zinkfingern befassen, wird in der vorliegenden Erfindung ausschließlich mit chemisch synthetisierten und in-vitro ligierten Peptiden gearbeitet. **(**US645324251**,** US20020061512A1**,** US20030059767A1**,** W00185765A2, US20040224385A1**,** US20040253623A1**,** US20020165356A1), und die Beschreibungen decken hierbei auch nicht den gesamten beliebigen Bereich der freien Variabilität der 64 in der vorliegenden Erfindung beschriebenen Möglichkeiten ab.

Die Zinkfinger in der vorliegenden Erfindung stellen DNA-Sonden dar, die unabhängig von biologischen Zusammenhängen an jedwede doppelsträngige DNA binden sollen. Sie können, analog zu Oligonukleotiden die an Einzelstrang-DNA hybridisieren, zur Detektion von beliebegen doppelsträngigen DNA-Fragmente z. B. an DNA-Arrays auf Bioichips dienen, aber natürlich auch in den schon beschriebenen Bereichen Verwendung finden könnten. Die schon geschützten, auch "polydaktylen" Zinkfinger sind ausnahmslos rekombinant, d. h. über den Umweg der Klonierung von Vektoren und Expression in geeigneten Wirtszellen, also *in vivo* erzeugt **(**US20030044809A1**,** W09942474A2**,** W09632475A2**,** WO9853059A1**).** Wenn so auch die gewüschte freie Kombinierbarkeit der einzelneen C2H2-Zinkfinger-Motive gegeben ist, so zeigt sich im Unterschied der Herstellung der Moleküle auch schon der Vorteil des hier beschriebenen Systems. Das aufwendige Klonieren mit all seinen notwendigen Präparationen und Verifikationen wird durch die Synthese von Peptid-Bibliotheken (Libraries) ersetzt, bei der die Qualitätskontrolle bereits während der Synthese erfolgt. Zudem ist die Zeitersparnis enorm. Eine neue Modifikation in der angewandten Methodik einer schon beschriebenen Peptidligation *(*Bang D, Kent SB. 2004. A one-pot total synthesis of crambin. Angew. Chem. Int- Ed. Engl. 43(19):2534-8*.),* bei der die Ligation von maximal drei Peptiden beschrieben ist, erlaubt uns die Ligation beliebig vieler Zinkfinger-Einzelmoleküle zur Detektion beliebig langer DNA-Doppelstrangfragmente.

Auch die schon beschrieben und geschützten Zinkfinger-Bibliotheken beruhen auf nicht chemisch synthetisierten Polypeptiden. **(**US20030165997A1**,** US20020146691A1**,** W02003066828A2**)**
Zu alle dem sind die hier beschriebenen Erkennungsmotive im alphahelikalen Teil der Zinkfingerpeptide (Position -1 bis 6) aus einem Vergleich von einer Vielzahl von Publikationen generiert und entsprechen an jeder Position der am häufigsten beschriebenen Konsensus-Aminosäure aus allen zur Verfügung gestandenen Publikationen. Diese unsere Motive entsprechen, zusätzlich zu der unterschiedlichen Generierung mittels chemischer Synthese, in ihrer Sequenz dadurch größtenteils nicht den schon in Patenten geschützen Motivsequenzen (siehe dort, bzw. vergl. Anhang).

In der alphahelikalen Bindedomäne des C2H2-Motivs wurden durch Extrapolation von Literaturdaten sämtliche theoretischen Aminosäuresequenzvarianten ermittelt, die quantitativ alle denkbar möglichen Basentriplette komplementieren können. Allen sich daraus ergebenden 64 (4³) Möglichkeiten wurde eine C2H2-Zinkfingermotiv-Aminosäuresequenz zugeordnet. Aus den sich hieraus ergebenden Peptidsequenzen wurde eine Liste erstellt, die im Anhang aufgeführt ist. Als Basis für die Aneinanderkopplung (Ligation resp. "Native PeptidLigation": Yeo, D. S. Y., Srinivasan, R., Grace, Y. J. C and S. Q Jao. 2004. Expanded Utilities of Native Chemical Ligation. Chem. Eur. J. 10:4664-4672*;* Harwig CW, Hoffman TZ, Wentworth AD, Janda KD 2000. A one-pot multistep approach to alpha-azido-phosphonate and phosphonothioate diesters: key intermediates in the synthesis of haptens for the generation of antibody ligases. Bioorg. Med. Chem. Lett. 1;10(9):915-918*)* der Zinkfingermotive dienen drei erstellte unterschiedliche Peptid-Bibliotheken (Bibliothek 1-3), jeweils mit den 64 möglichen Aminosäure-Sequenzkonstrukten (s. o. bzw. Anhang).

Die erste Bibliothek (Bibliothek 1) besteht aus den genannten 64 synthetischen Peptiden die jeweils am Aminoterminus eine Funktionalisierung (Bsp.: Fluorophore, Biotin) tragen. Dadurch sind sie am Aminoterminus geschützt und tragen gleichzeitig schon die (Farb-) Markierung, die letztendlich beispielsweise eine DNA-Sonde kennzeichnet.

Am Carboxyterminus tragen die Peptide die ersten zwei Aminosäuren des Folgepetids der Ligation. Dieses Folgepeptid beginnt aus ligationstechnischen Gründen mit dem 1. Cystein (siehe Skizze C2H2-Motiv), benötigt aber für eine funktionelle Ausbildung der Zinkfingerstruktur eben diese zwei Aminosäuren. Diese werden hier vom vorangestellten Ligationspartner (s. o.) so bereitgestellt.
Die Aminosäure des absoluten Carboxyterminus enthält einen Thioester.Am Carboxyterminus können die Peptide zusätzlich einen Spacer (Abstandshalter) tragen, der seine Funktion durch Ligation mit einem zweiten Zinkfingerpeptid erhält. Dieser Abstandshalter kann die Freiheitsgrade der einzelnen Zinkfingerpeptide innerhalb des Ligationsproduktes erhöhen.

Die 64 synthetischen Peptide der zweiten Bibliothek (Bibliothek 2) beginnen aminoterminal mit ihrem ersten Cystein; dieses Cystein ist durch eine Schutzgruppe (THZ: Bang D, Kent SB. 2004. A one-pot total synthesis of crambin. Angew. Chem. Int. Ed. Engl. 43(19):2534-8*.)* inaktiviert. Am carboxyterminalen Ende kann ein Linker angefügt sein (s. o.), sowie die ersten beiden Aminosäuren des jeweils folgenden C2H2-Motivs vor dem ersten Cystein (s. o.). Die Aminosäure des absoluten Carboxyterminus enthält einen Thioester.

Die 64 synthetischen Peptide der dritten Bibliothek (Bibliothek 3) beginnen aminoterminal mit ihrem ersten Cystein; dieses Cystein ist nicht geschützt.

Durch unterschiedlich kombinierte Ligationen aus den drei aufgeführten Bibliotheken kann jede denkbare DNA-Sequenz aus einem Vielfachen von drei mit den C2H2-Peptidsondenkonstrukten angesprochen werden.

Die so chemisch synthetisierten Polyzinkfingermolekülbinder zeichnen sich dadurch aus, dass sie in freier Kombination und in Form von Multimeren an jede beliebige Doppelstrang-DNA binden und dass dieser Doppelstrang-DNA-Abschnitt frei wählbar ist.

Das erfindungsgemäße Ligationsverfahren umfasst dabei folgende Schritte:
a)Ein Molekül(synth. Zinkfingerpeptid) aus Bibliothek 3 (ungeschütztes Cystein, kein Thioester am Carboxylende) wird mit einem Molekül aus Bibliothek II (geschütztes Cystein am Aminoterminus, Thioester am Carboxylende) über das freie Cystein mit dem Thioester ligiert; b)Entschützen des Cysteins (ursprünglich aus Bibliothek 2 stammend) dieses in Schritt a) entstandenen Doppelmoleküls;
c) Ligation dieses modifizierten Doppelmoleküls mit einem weiteren Molekül aus Bibliothek II über das nun freie Cystein mit dem Thioester des dritten Moleküls zu einem Polypeptid mit drei Zinkfingerpeptiden;
d) Entschützen des am Aminoterminus befindliche Cystein dieses in Schritt c entstandenen Moleküls;
e) Hinzufügen eines weiteren Moleküls aus Bibliothek II durch Ligation wie in Schritt c) beschreiben;
f) Wiederholen von Schritt e) bis die gewünschte Anzahl von C2H2-zinkfingerpeptiden erreicht ist
g) Finale Ligation mit einem Molekül aus Bibliothek I.
   Durch Schritt g) wird dem gewünschten Endmolekül eine Funktionalisierung hinzugefügt.

Die erfindungsgemäßen Polyzinkfingermolekülbindern finden Anwendung in der molekularen Analytik, z.B. als Sonden in der DNA-Analytik oder auch als Transkritptionsfaktoren. Vorstellbar für Analytikanwendungen sind auch Biochips, auf denen die erfindungsgemäßen Polyzinkfingermolekülbindern immobilisiert sind.

### Beispiel (siehe Abb. 2 in Verbindung mit Bibliotheken 1-3, Anhang):

Um einen DNA-Abschnitt von 15 Basenpaaren ansprechen zu können, wird ein Protein aus 5 C2H2-Zinkfingermotiven benötigt. Die serielle Ligation beginnt vom Carboxyterminus aus. Bei der Ligation ist darauf zu achten, dass sich die Peptide revers mit der DNA verbinden, d. h. die letzte Aminosäure (Carboxyterminus) des gesamten Peptids liegt an der ersten Base (5'-Ende) des DNA-Abschnitts an. Ein entsprechender Zinkfinger aus der 3.

Bibliothek der den ersten drei Basenpaaren des gewählten DNA-Abschnittes entspricht, wird mit einem Motiv, das dem nachfolgenden Triplett entspricht, aus Bibliothek zwei ligiert, wobei (s. o.) die aminoterminale Aminosäure die 3'-Base des Tripletts bindet und die carboxyterminale Aminosäure die 5'-Base.

In einem zweiten Schritt werden, vom 5'-Ende des DNA-Abschnittes gesehen, das dritte und das vorletzte Triplett bzw. die korrespondierenden Peptide ligiert.

Die aminoterminalen Cysteine verbinden sich jeweils mit der carboxyterminalen Thioester des Peptidpartners. Hierbei wird das geschützte Cysteine des ersten Peptidpaares entschützt und auf die wie schon beschriebene Weise mit dem Thioester des dritten Peptids ligiert werden. In diesem Beispiel findet nach einer weiteren Cystein/Thioester-Bindung des vorletzten Peptids, die finale Ligation mit dem Fluorophoren-tragenden Peptid statt, indem das noch geschützte Cysteine des o. g. Viererblocks entschützt wird und am Thioester des letzten Peptids, das dem letzten Triplett des DNA-Abschnittes entspricht, ligiert wird. Man könnte zuvor noch weitere Peptide aus Bibliothek 2 anfügen anstatt des hier abschließenden Fluorophorenpeptids, um eine größere Anzahl an Basenpaaren anzusprechen (Vielfaches von drei).

ABBILDUNG 2:

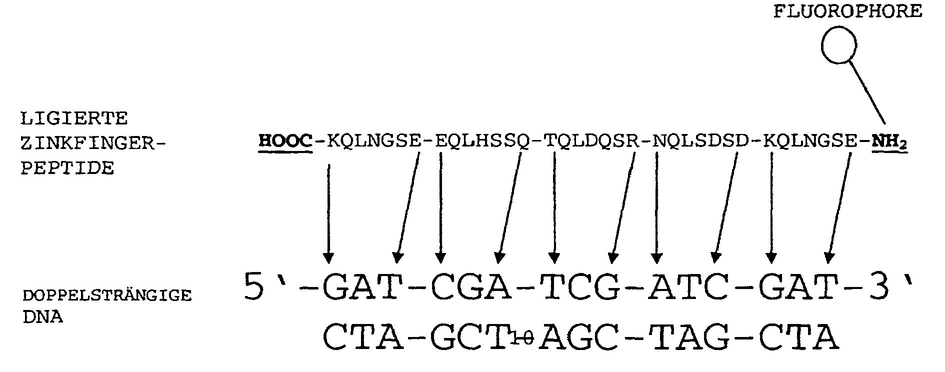

Die vorliegende Erfindung ist gekennzeichnet durch die Erstellung einer synthetischen, quantitativen Peptid-Bibliothek von Zinkfingermotiven des C2H2-Typs. Hierbei wird die Bindedomäne des DNA-Triplett-bindenden alphahelikalen Bereiches des Peptids in ihrer Aminosäuresequenz derart variiert, dass ein theoretisches Binden an allen 64 (4³) möglichen Triplettvarianten erfolgen kann.

Ein weiteres Kennzeichen dieser Erfindung ist die Methodik der Ligation der einzelnen o. g. Peptidvariationen, die es erlaubt, beliebig viele synthetische Peptide derart zusammenzufügen, dass jede beliebige Basenpaarabfolge eines als ein Vielfaches von drei (s. o., Basentripletts) gewählten Sequenzabschnittes auf einer doppelsträngigen DNA adressiert werden kann. Diese Methodik beinhaltet die in der hier beschriebenen Art konstruierten Modifikationen der Peptide, die eine sinnvolle und funktionale Ligation der Einzelpeptide zulässt (siehe Bibliotheken 1-3).

Das Hauptalleinstellungsmerkmal der vorliegenden Erfindung ist ein Verfahren zur Herstellung von an beliebigen doppelsträngigen DNA-Abschnitten bindenden Sonden auf der Basis von ligierten, synthetischen Zinkfingerpeptiden.

Neu an der Erfindung ist die Konstruktion variabler Sonden (für die Detektion beispielsweise mit Fluorophoren markiert) in Form von Zinkfinger-Peptiden an doppelsträngige DNA-Abschnitte jedweder Sequenz. Durch freie Kombination und Ligation einzelner C2H2-Zinkfinger bzw. Zinkfinger-Motive kann jede beliebige frei wählbare DNA-Sequenz im Doppelstrang mit diesen Peptid-Sonden spezifisch detektiert werden. Dies ist in Analogie zu Oligonukleotidsonden an einzelsträngige DNA zu betrachten, ohne die Schwierigkeit zunächst eben diese Einzelstränge zu generieren.

In diesem Zusammenhang insbesondere neu sind die chemisch synthetisierten Zinkfinger-Peptide, sowie die frei kombinierbare Ligation der einzelnen C2H2-Motive (Building Blocks) zu multiplen Zinkfingerkonkatameren. Dies ist sinnvoll für Ligationsprodukte von bis zu 10meren, also 10 ligierten Zinkfingermolekülen, das einer spezifischen Sonde für ein 30 Basenpaar-Sequenzfragment entspricht, ist aber auch für noch längere DNA-Abschnitte denkbar. In der Literatur ist eine in vitro-Ligation von Peptiden mit bis zu maximal drei Molekülen beschrieben (siehe: Bang, D. and Kent, S. B. 2004).

Das Verändernde der Erfindung an schon beschriebenen Verfahren ist die chemische Synthese der Zinkfingerpeptide und die Ligation zu beliebig wählbaren DNA-Sonden im Gegensatz zu beschriebenen Verfahren (alle gelisteten Patente, insbesondere die Bibliotheken), bei denen die DNA-Sequenzen, die zu den Zinkfingerpeptiden führen, erst in entsprechend gewünschter Anzahl für entsprechend gewünschte Motive hintereinander in Expressions-Vektoren kloniert werden müssen, um anschließend die gewünschten Zinkfinger-Moleküle gentechnisch in geeigneten Wirtszellen exprimieren zu können.

Die neuen technischen Verfahren zur kommerziellen Synthese von Peptiden, rückt die Synthese der erforderlichen Peptide in einen vergleichbaren finanziellen Rahmen und ist aber ungleich zeitsparender und genauer. Durch das vorgestellte Ligationsverfahren können die beschrieben Peptid-Bibliotheken, einmal synthetisiert, vorgehalten werden, und die Sonden auf jede beliebige Doppelstrang-DNA-Sequenz, in kürzester Zeit zusammenligiert werden, ohne eben ein erneutes Expressionsverfahren mit all seinen Klonierungen und Kontrollen etc. in Gang setzen zu müssen.

Zudem ist die freie Wählbarkeit der doppelsträngigen DNA-Sequnzen gegeben. Im Gegensatz zu PNA-Sonden, die zwar auch an doppelsträngiger DNA binden, aber unabdingbare Spezifikationen an die DNA-Sequenzen erfordern (Nielsen, P.E., 2005. Gene targeting using peptide nucleic acid. Methods Mol. Biol. 288:343-58), welches die freie Wählbarkeit der zu detektierenden DNA-Abschnitte stark einschränkt, können hierbei nicht alle denkbaren DNA-Sequenzen frei wählbar angesprochen werden. Zudem ist die Synthese der PNA erheblich teurer, da nur wenige Laboratorien in der Lage sind diese herzustellen (Einzelanfertigungen).

SEQUENCE LISTING

<110> Fraunhofer-Gesellschaft zur Förderung der angewandten
      Forschung e.V.

<120> Ligation synthetischer Zinkfinger-Peptide zu seriellen
      Bindeproteinen für die spezifische Adressierung doppelstrangiger
      DNA-Bereiche (Zinkfinger-Sonden)

<130> L 2725 EP

<160> 128

<170> PatentIn version 3.3

<210> 1
<211> 32
<212> PRT
<213> artificial sequence

<220>
<223> C2H2-zinc-finger-motive


<220>
<221> misc_feature
<222> (1)..(1)
<223> Xaa can be any naturally occurring amino acid

<220>
<221> misc_feature
<222> (2)..(2)
<223> hydrophobic amino acid

<220>
<221> misc_feature
<222> (3)..(3)
<223> Xaa can be any naturally occurring amino acid

<220>
<221> misc_feature
<222> (5)..(7)
<223> Xaa can be any naturally occurring amino acid

<220>
<221> misc_feature
<222> (9)..(11)
<223> Xaa can be any naturally occurring amino acid

<220>
<221> misc_feature
<222> (12)..(12)
<223> hydrophobic amino acid

<220>
<221> misc_feature
<222> (13)..(13)
<223> Xaa can be any naturally occurring amino acid

<220>
<221> misc_feature
<222> (22)..(24)
<223> Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  1

Xaa Xaa Xaa Cys Xaa Xaa Xaa Cys Xaa Xaa Xaa Xaa Xaa Arg Ser Gln
1               5                   10                  15

Asp Leu Gln Lys His Xaa Xaa Xaa His Xaa Xaa Xaa Xaa Xaa Xaa Xaa
            20                  25                  30

<210>  2
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  2

Xaa Xaa Xaa Cys Xaa Xaa Xaa Cys Xaa Xaa Xaa Xaa Xaa Gln Ser Ser
1               5                   10                  15


Asn Leu Gln Asn His Xaa Xaa Xaa His Xaa Xaa Xaa Xaa Xaa Xaa Xaa
            20                  25                  30


<210>  3
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  3

Xaa Xaa Xaa Cys Xaa Xaa Xaa Cys Xaa Xaa Xaa Xaa Xaa Arg Ser Ser
1               5                   10                  15


His Leu Gln Lys His Xaa Xaa Xaa His Xaa Xaa Xaa Xaa Xaa Xaa Xaa
            20                  25                  30


<210>  4
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  4

Xaa Xaa Xaa Cys Xaa Xaa Xaa Cys Xaa Xaa Xaa Xaa Xaa Gln Ser Ser
1               5                   10                  15

His Leu Gln Lys His Xaa Xaa Xaa His Xaa Xaa Xaa Xaa Xaa Xaa Xaa
            20                  25                  30

<210>  5
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  5

Xaa Xaa Xaa Cys Xaa Xaa Xaa Cys Xaa Xaa Xaa Xaa Xaa Asp Ser Ser
1               5                   10                  15


His Leu Gln Lys His Xaa Xaa Xaa His Xaa Xaa Xaa Xaa Xaa Xaa Xaa
            20                  25                  30

<210>  6
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  6

Xaa Xaa Xaa Cys Xaa Xaa Xaa Cys Xaa Xaa Xaa Xaa Xaa Glu Ser Ser
1               5                   10                  15

His Leu Gln Lys His Xaa Xaa Xaa His Xaa Xaa Xaa Xaa Xaa Xaa Xaa
            20                  25                  30

<210>  7
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  7

Xaa Xaa Xaa Cys Xaa Xaa Xaa Cys Xaa Xaa Xaa Xaa Xaa Arg Ser Ser
1               5                   10                  15

His Leu Gln Asn His Xaa Xaa Xaa His Xaa Xaa Xaa Xaa Xaa Xaa Xaa
            20                  25                  30

<210>  8
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221> misc_feature
<222> (22)..(24)
<223> Xaa can be any naturally occurring amino acid

<220>
<221> misc_feature
<222> (26)..(30)
<223> Xaa can be any naturally occurring amino acid

<220>
<221> misc_feature
<222> (31)..(31)
<223> hydrophobic amino acid

<220>
<221> misc_feature
<222> (32)..(32)
<223> Xaa can be any naturally occurring amino acid

<400> 8

Xaa Xaa Xaa Cys Xaa Xaa Xaa Cys Xaa Xaa Xaa Xaa Xaa Arg Ser Ser
1               5                   10                  15

His Leu Gln Thr His Xaa Xaa Xaa His Xaa Xaa Xaa Xaa Xaa Xaa Xaa
            20                  25                  30

<210> 9
<211> 32
<212> PRT
<213> artificial sequence

<220>
<223> C2H2-zinc-finger-motive

<220>
<221> misc_feature
<222> (1)..(1)
<223> Xaa can be any naturally occurring amino acid

<220>
<221> misc_feature
<222> (2)..(2)
<223> hydrophobic amino acid

<220>
<221> misc_feature
<222> (3)..(3)
<223> Xaa can be any naturally occurring amino acid

<220>
<221> misc_feature
<222> (5)..(7)
<223> Xaa can be any naturally occurring amino acid

<220>
<221> misc_feature
<222> (9)..(11)
<223> Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  9

Xaa Xaa Xaa Cys Xaa Xaa Xaa Cys Xaa Xaa Xaa Xaa Xaa Arg Ser Ser
1               5                   10                  15

His Leu Gln Glu His Xaa Xaa Xaa His Xaa Xaa Xaa Xaa Xaa Xaa Xaa
            20                  25                  30

<210>  10
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  10

Xaa Xaa Xaa Cys Xaa Xaa Xaa Cys Xaa Xaa Xaa Xaa Xaa Arg Ser Gly
1               5                   10                  15

Asn Leu Gln Asn His Xaa Xaa Xaa His Xaa Xaa Xaa Xaa Xaa Xaa Xaa
            20                  25                  30

<210>  11
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  11

Xaa Xaa Xaa Cys Xaa Xaa Xaa Cys Xaa Xaa Xaa Xaa Xaa Glu Ser Gly
1               5                   10                  15

Asn Leu Gln Asn His Xaa Xaa Xaa His Xaa Xaa Xaa Xaa Xaa Xaa Xaa
            20                  25                  30

<210>  12
<211>  32
<212>  PRT

<213>  artificial sequence

<220>
<223>  C2H2-zinc-finger-motive



<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  12

Xaa Xaa Xaa Cys Xaa Xaa Xaa Cys Xaa Xaa Xaa Xaa Xaa Asp Ser Gly

1                   5                   10                  15

Asn Leu Gln Asn His Xaa Xaa Xaa His Xaa Xaa Xaa Xaa Xaa Xaa Xaa
            20                  25                  30

<210>  13
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)

<223>  hydrophobic amino acid

<220>
<221>  misc_feature
<222>  (32)..(32)
<223>  Xaa can be any naturally occurring amino acid

<400>  13

Xaa Xaa Xaa Cys Xaa Xaa Xaa Cys Xaa Xaa Xaa Xaa Xaa Gln Ser Gly
1               5                   10                  15

Asn Leu Gln Lys His Xaa Xaa Xaa His Xaa Xaa Xaa Xaa Xaa Xaa Xaa
            20                  25                  30

<210>  14
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)

<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  14

Xaa Xaa Xaa Cys Xaa Xaa Xaa Cys Xaa Xaa Xaa Xaa Xaa Gln Ser Gly
1               5                   10                  15


Asn Leu Gln Thr His Xaa Xaa Xaa His Xaa Xaa Xaa Xaa Xaa Xaa Xaa
            20                  25                  30


<210>  15
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)

<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  15

Xaa Xaa Xaa Cys Xaa Xaa Xaa Cys Xaa Xaa Xaa Xaa Xaa Gln Ser Gly
1               5                   10                  15


Asn Leu Gln Glu His Xaa Xaa Xaa His Xaa Xaa Xaa Xaa Xaa Xaa Xaa
            20                  25                  30


<210>  16
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)

<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  16

Xaa Xaa Xaa Cys Xaa Xaa Xaa Cys Xaa Xaa Xaa Xaa Xaa Glu Ser Asp
1               5                   10                  15

Ser Leu Gln Thr His Xaa Xaa Xaa His Xaa Xaa Xaa Xaa Xaa Xaa Xaa
            20                  25                  30

<210>  17
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)

<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  17

Xaa Xaa Xaa Cys Xaa Xaa Xaa Cys Xaa Xaa Xaa Xaa Xaa Arg Ser Asp
1               5                   10                  15

Ser Leu Gln Thr His Xaa Xaa Xaa His Xaa Xaa Xaa Xaa Xaa Xaa Xaa
            20                  25                  30

<210>  18
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  18

Xaa Xaa Xaa Cys Xaa Xaa Xaa Cys Xaa Xaa Xaa Xaa Xaa Gln Ser Asp
1               5                   10                  15

Ser Leu Gln Thr His Xaa Xaa Xaa His Xaa Xaa Xaa Xaa Xaa Xaa Xaa

            20                  25                  30

<210> 19
<211> 32
<212> PRT
<213> artificial sequence

<220>
<223> C2H2-zinc-finger-motive


<220>
<221> misc_feature
<222> (1)..(1)
<223> Xaa can be any naturally occurring amino acid

<220>
<221> misc_feature
<222> (2)..(2)
<223> hydrophobic amino acid

<220>
<221> misc_feature
<222> (3)..(3)
<223> Xaa can be any naturally occurring amino acid

<220>
<221> misc_feature
<222> (5)..(7)
<223> Xaa can be any naturally occurring amino acid

<220>
<221> misc_feature
<222> (9)..(11)
<223> Xaa can be any naturally occurring amino acid

<220>
<221> misc_feature
<222> (12)..(12)
<223> hydrophobic amino acid

<220>
<221> misc_feature
<222> (13)..(13)
<223> Xaa can be any naturally occurring amino acid

<220>
<221> misc_feature
<222> (22)..(24)
<223> Xaa can be any naturally occurring amino acid

<220>
<221> misc_feature
<222> (26)..(30)
<223> Xaa can be any naturally occurring amino acid

<220>
<221> misc_feature
<222> (31)..(31)
<223> hydrophobic amino acid

<220>
<221> misc_feature

<222>  (32)..(32)
<223>  Xaa can be any naturally occurring amino acid

<400>  19

Xaa Xaa Xaa Cys Xaa Xaa Xaa Cys Xaa Xaa Xaa Xaa Xaa Asp Ser Asp
1               5                   10                  15

Ser Leu Gln Thr His Xaa Xaa Xaa His Xaa Xaa Xaa Xaa Xaa Xaa Xaa
            20                  25                  30

<210>  20
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature

<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  20

Xaa Xaa Xaa Cys Xaa Xaa Xaa Cys Xaa Xaa Xaa Xaa Xaa Glu Ser Asp
1               5                   10                  15

Ser Leu Gln Lys His Xaa Xaa Xaa His Xaa Xaa Xaa Xaa Xaa Xaa Xaa
            20                  25                  30

<210>  21
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature

<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  21

Xaa Xaa Xaa Cys Xaa Xaa Xaa Cys Xaa Xaa Xaa Xaa Xaa Glu Ser Asp
1               5                   10                  15


Ser Leu Gln Asn His Xaa Xaa Xaa His Xaa Xaa Xaa Xaa Xaa Xaa Xaa
            20                  25                  30


<210>  22
<211>  32
<212>  PRT
<213>  artificial sequence

<220>
<223>  C2H2-zinc-finger-motive



<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature

<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  22

Xaa Xaa Xaa Cys Xaa Xaa Xaa Cys Xaa Xaa Xaa Xaa Xaa Glu Ser Asp
1               5                   10                  15


Ser Leu Gln Glu His Xaa Xaa Xaa His Xaa Xaa Xaa Xaa Xaa Xaa Xaa
            20                  25                  30


<210>  23
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature

<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  23

Xaa Xaa Xaa Cys Xaa Xaa Xaa Cys Xaa Xaa Xaa Xaa Xaa Asp Ser Gln
1               5                   10                  15

Asp Leu Gln Glu His Xaa Xaa Xaa His Xaa Xaa Xaa Xaa Xaa Xaa Xaa
            20                  25                  30

<210>  24
<211>  32
<212>  PRT
<213>  artificial sequence

<220>
<223>  C2H2-zinc-finger-motive

<220>
<221>  misc_feature

<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  24

Xaa Xaa Xaa Cys Xaa Xaa Xaa Cys Xaa Xaa Xaa Xaa Xaa Arg Ser Gln
1               5                   10                  15

Asp Leu Gln Glu His Xaa Xaa Xaa His Xaa Xaa Xaa Xaa Xaa Xaa Xaa
            20                  25                  30

<210>  25

<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  25

Xaa Xaa Xaa Cys Xaa Xaa Xaa Cys Xaa Xaa Xaa Xaa Xaa Gln Ser Gln
1               5                   10                  15

Asp Leu Gln Glu His Xaa Xaa Xaa His Xaa Xaa Xaa Xaa Xaa Xaa Xaa
            20                  25                  30

<210>  26
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>

<221>  misc_feature
<222>  (31)..(31)
<223>  hydrophobic amino acid

<220>
<221>  misc_feature
<222>  (32)..(32)
<223>  Xaa can be any naturally occurring amino acid

<400>  26

Xaa Xaa Xaa Cys Xaa Xaa Xaa Cys Xaa Xaa Xaa Xaa Xaa Glu Ser Gln
1               5                   10                  15

Asp Leu Gln Glu His Xaa Xaa Xaa His Xaa Xaa Xaa Xaa Xaa Xaa Xaa
            20                  25                  30

<210>  27
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>

<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  27

Xaa Xaa Xaa Cys Xaa Xaa Xaa Cys Xaa Xaa Xaa Xaa Xaa Asp Ser Gln
1               5                   10                  15

Asp Leu Gln Lys His Xaa Xaa Xaa His Xaa Xaa Xaa Xaa Xaa Xaa Xaa
            20                  25                  30

<210>  28
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>

<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  28

Xaa Xaa Xaa Cys Xaa Xaa Xaa Cys Xaa Xaa Xaa Xaa Xaa Asp Ser Gln
1               5                   10                  15

Asp Leu Gln Asn His Xaa Xaa Xaa His Xaa Xaa Xaa Xaa Xaa Xaa Xaa
            20                  25                  30

<210>  29
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>

<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  29

Xaa Xaa Xaa Cys Xaa Xaa Xaa Cys Xaa Xaa Xaa Xaa Xaa Asp Ser Gln
1               5                   10                  15

Asp Leu Gln Thr His Xaa Xaa Xaa His Xaa Xaa Xaa Xaa Xaa Xaa Xaa
            20                  25                  30

<210>  30
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>

<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  30

Xaa Xaa Xaa Cys Xaa Xaa Xaa Cys Xaa Xaa Xaa Xaa Xaa Gln Ser Gln
1               5                   10                  15

Asp Leu Gln Lys His Xaa Xaa Xaa His Xaa Xaa Xaa Xaa Xaa Xaa Xaa
            20                  25                  30

<210>  31
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  31

Xaa Xaa Xaa Cys Xaa Xaa Xaa Cys Xaa Xaa Xaa Xaa Xaa Glu Ser Gln
1               5                   10                  15

Asp Leu Gln Lys His Xaa Xaa Xaa His Xaa Xaa Xaa Xaa Xaa Xaa Xaa
            20                  25                  30

<210>  32
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  32

Xaa Xaa Xaa Cys Xaa Xaa Xaa Cys Xaa Xaa Xaa Xaa Xaa Arg Ser Gly
1               5                   10                  15

Asn Leu Gln Lys His Xaa Xaa Xaa His Xaa Xaa Xaa Xaa Xaa Xaa Xaa
            20                  25                  30

<210>  33
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  33

Xaa Xaa Xaa Cys Xaa Xaa Xaa Cys Xaa Xaa Xaa Xaa Xaa Glu Ser Gly
1               5                   10                  15


Asn Leu Gln Lys His Xaa Xaa Xaa His Xaa Xaa Xaa Xaa Xaa Xaa Xaa
            20                  25                  30


<210>  34
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  34

Xaa Xaa Xaa Cys Xaa Xaa Xaa Cys Xaa Xaa Xaa Xaa Xaa Asp Ser Gly
1               5                   10                  15

Asn Leu Gln Lys His Xaa Xaa Xaa His Xaa Xaa Xaa Xaa Xaa Xaa Xaa
            20                  25                  30

<210>  35
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  35

Xaa Xaa Xaa Cys Xaa Xaa Xaa Cys Xaa Xaa Xaa Xaa Xaa Arg Ser Asp
1               5                   10                  15


Ser Leu Gln Lys His Xaa Xaa Xaa His Xaa Xaa Xaa Xaa Xaa Xaa Xaa
            20                  25                  30


<210>  36
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  36

Xaa Xaa Xaa Cys Xaa Xaa Xaa Cys Xaa Xaa Xaa Xaa Xaa Gln Ser Asp
1               5                   10                  15

Ser Leu Gln Lys His Xaa Xaa Xaa His Xaa Xaa Xaa Xaa Xaa Xaa Xaa
            20                  25                  30

<210>  37
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  37

Xaa Xaa Xaa Cys Xaa Xaa Xaa Cys Xaa Xaa Xaa Xaa Xaa Asp Ser Asp
1               5                   10                  15

Ser Leu Gln Lys His Xaa Xaa Xaa His Xaa Xaa Xaa Xaa Xaa Xaa Xaa
            20                  25                  30

<210>  38
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  38

Xaa Xaa Xaa Cys Xaa Xaa Xaa Cys Xaa Xaa Xaa Xaa Xaa Gln Ser Ser
1               5                   10                  15

His Leu Gln Asn His Xaa Xaa Xaa His Xaa Xaa Xaa Xaa Xaa Xaa Xaa
            20                  25                  30

<210>  39
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221> misc_feature
<222> (31)..(31)
<223> hydrophobic amino acid

<220>
<221> misc_feature
<222> (32)..(32)
<223> Xaa can be any naturally occurring amino acid

<400> 39

Xaa Xaa Xaa Cys Xaa Xaa Xaa Cys Xaa Xaa Xaa Xaa Xaa Gln Ser Gln
1               5                   10                  15

Asp Leu Gln Asn His Xaa Xaa Xaa His Xaa Xaa Xaa Xaa Xaa Xaa Xaa
            20                  25                  30

<210> 40
<211> 32
<212> PRT
<213> artificial sequence

<220>
<223> C2H2-zinc-finger-motive

<220>
<221> misc_feature
<222> (1)..(1)
<223> Xaa can be any naturally occurring amino acid

<220>
<221> misc_feature
<222> (2)..(2)
<223> hydrophobic amino acid

<220>
<221> misc_feature
<222> (3)..(3)
<223> Xaa can be any naturally occurring amino acid

<220>
<221> misc_feature
<222> (5)..(7)
<223> Xaa can be any naturally occurring amino acid

<220>
<221> misc_feature
<222> (9)..(11)
<223> Xaa can be any naturally occurring amino acid

<220>
<221> misc_feature
<222> (12)..(12)
<223> hydrophobic amino acid

<220>
<221> misc_feature
<222> (13)..(13)
<223> Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  40

Xaa Xaa Xaa Cys Xaa Xaa Xaa Cys Xaa Xaa Xaa Xaa Xaa Gln Ser Asp
1               5                   10                  15


Ser Leu Gln Asn His Xaa Xaa Xaa His Xaa Xaa Xaa Xaa Xaa Xaa Xaa
            20                  25                  30


<210>  41
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  41

Xaa Xaa Xaa Cys Xaa Xaa Xaa Cys Xaa Xaa Xaa Xaa Xaa Glu Ser Gln
1               5                   10                  15

Asp Leu Gln Asn His Xaa Xaa Xaa His Xaa Xaa Xaa Xaa Xaa Xaa Xaa
            20                  25                  30

<210>  42
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  42

Xaa Xaa Xaa Cys Xaa Xaa Xaa Cys Xaa Xaa Xaa Xaa Xaa Arg Ser Gln
1               5                   10                  15


Asp Leu Gln Asn His Xaa Xaa Xaa His Xaa Xaa Xaa Xaa Xaa Xaa Xaa
            20                  25                  30


<210>  43
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221> misc_feature
<222> (2)..(2)
<223> hydrophobic amino acid

<220>
<221> misc_feature
<222> (3)..(3)
<223> Xaa can be any naturally occurring amino acid

<220>
<221> misc_feature
<222> (5)..(7)
<223> Xaa can be any naturally occurring amino acid

<220>
<221> misc_feature
<222> (9)..(11)
<223> Xaa can be any naturally occurring amino acid

<220>
<221> misc_feature
<222> (12)..(12)
<223> hydrophobic amino acid

<220>
<221> misc_feature
<222> (13)..(13)
<223> Xaa can be any naturally occurring amino acid

<220>
<221> misc_feature
<222> (22)..(24)
<223> Xaa can be any naturally occurring amino acid

<220>
<221> misc_feature
<222> (26)..(30)
<223> Xaa can be any naturally occurring amino acid

<220>
<221> misc_feature
<222> (31)..(31)
<223> hydrophobic amino acid

<220>
<221> misc_feature
<222> (32)..(32)
<223> Xaa can be any naturally occurring amino acid

<400> 43

Xaa Xaa Xaa Cys Xaa Xaa Xaa Cys Xaa Xaa Xaa Xaa Xaa Asp Ser Asp
1               5                   10                  15

Ser Leu Gln Asn His Xaa Xaa Xaa His Xaa Xaa Xaa Xaa Xaa Xaa Xaa
            20                  25                  30

<210> 44
<211> 32
<212> PRT

<213>  artificial sequence

<220>
<223>  C2H2-zinc-finger-motive



<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  44

Xaa Xaa Xaa Cys Xaa Xaa Xaa Cys Xaa Xaa Xaa Xaa Xaa Asp Ser Ser

1                   5                   10                  15

His Leu Gln Asn His Xaa Xaa Xaa His Xaa Xaa Xaa Xaa Xaa Xaa Xaa
                20                  25                  30

<210>  45
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)

<223>  hydrophobic amino acid

<220>
<221>  misc_feature
<222>  (32)..(32)
<223>  Xaa can be any naturally occurring amino acid

<400>  45

Xaa Xaa Xaa Cys Xaa Xaa Xaa Cys Xaa Xaa Xaa Xaa Xaa Glu Ser Ser
1               5                   10                  15

His Leu Gln Asn His Xaa Xaa Xaa His Xaa Xaa Xaa Xaa Xaa Xaa Xaa
            20                  25                  30

<210>  46
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)

<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  46

Xaa Xaa Xaa Cys Xaa Xaa Xaa Cys Xaa Xaa Xaa Xaa Xaa Arg Ser Asp
1               5                   10                  15

Ser Leu Gln Asn His Xaa Xaa Xaa His Xaa Xaa Xaa Xaa Xaa Xaa Xaa
            20                  25                  30

<210>  47
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)

<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  47

Xaa Xaa Xaa Cys Xaa Xaa Xaa Cys Xaa Xaa Xaa Xaa Xaa Glu Ser Gly
1               5                   10                  15

Asn Leu Gln Thr His Xaa Xaa Xaa His Xaa Xaa Xaa Xaa Xaa Xaa Xaa
            20                  25                  30

<210>  48
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)

<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  48

Xaa Xaa Xaa Cys Xaa Xaa Xaa Cys Xaa Xaa Xaa Xaa Xaa Glu Ser Ser
1               5                   10                  15

His Leu Gln Thr His Xaa Xaa Xaa His Xaa Xaa Xaa Xaa Xaa Xaa Xaa
            20                  25                  30

<210>  49
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)

<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  49

Xaa Xaa Xaa Cys Xaa Xaa Xaa Cys Xaa Xaa Xaa Xaa Xaa Glu Ser Gln
1               5                   10                  15


Asp Leu Gln Thr His Xaa Xaa Xaa His Xaa Xaa Xaa Xaa Xaa Xaa Xaa
            20                  25                  30


<210>  50
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  50

Xaa Xaa Xaa Cys Xaa Xaa Xaa Cys Xaa Xaa Xaa Xaa Xaa Gln Ser Gln
1               5                   10                  15


Asp Leu Gln Thr His Xaa Xaa Xaa His Xaa Xaa Xaa Xaa Xaa Xaa Xaa

20                  25                  30

<210>  51
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature

<222>  (32)..(32)
<223>  Xaa can be any naturally occurring amino acid

<400>  51

Xaa Xaa Xaa Cys Xaa Xaa Xaa Cys Xaa Xaa Xaa Xaa Xaa Arg Ser Gln
1               5                   10                  15

Asp Leu Gln Thr His Xaa Xaa Xaa His Xaa Xaa Xaa Xaa Xaa Xaa Xaa
            20                  25                  30

<210>  52
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature

<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  52

Xaa Xaa Xaa Cys Xaa Xaa Xaa Cys Xaa Xaa Xaa Xaa Xaa Asp Ser Gly
1               5                   10                  15

Asn Leu Gln Thr His Xaa Xaa Xaa His Xaa Xaa Xaa Xaa Xaa Xaa Xaa
            20                  25                  30

<210>  53
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature

<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  53

Xaa Xaa Xaa Cys Xaa Xaa Xaa Cys Xaa Xaa Xaa Xaa Xaa Asp Ser Ser
1               5                   10                  15


His Leu Gln Thr His Xaa Xaa Xaa His Xaa Xaa Xaa Xaa Xaa Xaa Xaa
            20                  25                  30


<210>  54
<211>  32
<212>  PRT
<213>  artificial sequence

<220>
<223>  C2H2-zinc-finger-motive



<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature

<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  54

Xaa Xaa Xaa Cys Xaa Xaa Xaa Cys Xaa Xaa Xaa Xaa Xaa Arg Ser Gly
1               5                   10                  15

Asn Leu Gln Thr His Xaa Xaa Xaa His Xaa Xaa Xaa Xaa Xaa Xaa Xaa
            20                  25                  30

<210>  55
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature

<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  55

Xaa Xaa Xaa Cys Xaa Xaa Xaa Cys Xaa Xaa Xaa Xaa Xaa Gln Ser Ser
1               5                   10                  15


His Leu Gln Thr His Xaa Xaa Xaa His Xaa Xaa Xaa Xaa Xaa Xaa Xaa
            20                  25                  30


<210>  56
<211>  32
<212>  PRT
<213>  artificial sequence

<220>
<223>  C2H2-zinc-finger-motive


<220>
<221>  misc_feature

<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  56

Xaa Xaa Xaa Cys Xaa Xaa Xaa Cys Xaa Xaa Xaa Xaa Xaa Asp Ser Gly
1               5                   10                  15

Asn Leu Gln Glu His Xaa Xaa Xaa His Xaa Xaa Xaa Xaa Xaa Xaa Xaa
            20                  25                  30

<210>  57

<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  57

Xaa Xaa Xaa Cys Xaa Xaa Xaa Cys Xaa Xaa Xaa Xaa Xaa Asp Ser Asp
1               5                   10                  15

Ser Leu Gln Glu His Xaa Xaa Xaa His Xaa Xaa Xaa Xaa Xaa Xaa Xaa
            20                  25                  30

<210>  58
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>

<221>  misc_feature
<222>  (31)..(31)
<223>  hydrophobic amino acid

<220>
<221>  misc_feature
<222>  (32)..(32)
<223>  Xaa can be any naturally occurring amino acid

<400>  58

Xaa Xaa Xaa Cys Xaa Xaa Xaa Cys Xaa Xaa Xaa Xaa Xaa Asp Ser Ser
1               5                   10                  15


His Leu Gln Glu His Xaa Xaa Xaa His Xaa Xaa Xaa Xaa Xaa Xaa Xaa
            20                  25                  30


<210>  59
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>

<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  59

Xaa Xaa Xaa Cys Xaa Xaa Xaa Cys Xaa Xaa Xaa Xaa Xaa Gln Ser Ser
1               5                   10                  15

His Leu Gln Glu His Xaa Xaa Xaa His Xaa Xaa Xaa Xaa Xaa Xaa Xaa
            20                  25                  30

<210>  60
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>

<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  60

Xaa Xaa Xaa Cys Xaa Xaa Xaa Cys Xaa Xaa Xaa Xaa Xaa Glu Ser Ser
1               5                   10                  15

His Leu Gln Glu His Xaa Xaa Xaa His Xaa Xaa Xaa Xaa Xaa Xaa Xaa
            20                  25                  30

<210>  61
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>

<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  61

Xaa Xaa Xaa Cys Xaa Xaa Xaa Cys Xaa Xaa Xaa Xaa Xaa Arg Ser Gly
1               5                   10                  15


Asn Leu Gln Glu His Xaa Xaa Xaa His Xaa Xaa Xaa Xaa Xaa Xaa Xaa
            20                  25                  30


<210>  62
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>

<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  62

Xaa Xaa Xaa Cys Xaa Xaa Xaa Cys Xaa Xaa Xaa Xaa Xaa Glu Ser Gly
1               5                   10                  15

Asn Leu Gln Glu His Xaa Xaa Xaa His Xaa Xaa Xaa Xaa Xaa Xaa Xaa
            20                  25                  30

<210>  63
<211>  32
<212>  PRT
<213>  artificial sequence

<220>
<223>  C2H2-zinc-finger-motive


<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  63

Xaa Xaa Xaa Cys Xaa Xaa Xaa Cys Xaa Xaa Xaa Xaa Xaa Gln Ser Asp
1               5                   10                  15

Ser Leu Gln Glu His Xaa Xaa Xaa His Xaa Xaa Xaa Xaa Xaa Xaa Xaa
            20                  25                  30

<210>  64
<211>  32
<212>  PRT
<213>  artificial sequence

<220>
<223>  C2H2-zinc-finger-motive

<220>
<221>  misc_feature
<222>  (1)..(1)
<223>  Xaa can be any naturally occurring amino acid

<220>
<221>  misc_feature
<222>  (2)..(2)
<223>  hydrophobic amino acid

<220>
<221>  misc_feature
<222>  (3)..(3)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(11)
<223>  Xaa can be any naturally occurring amino acid

<220>
<221>  misc_feature
<222>  (12)..(12)
<223>  hydrophobic amino acid

<220>
<221>  misc_feature
<222>  (13)..(13)
<223>  Xaa can be any naturally occurring amino acid

<220>
<221>  misc_feature
<222>  (22)..(24)
<223>  Xaa can be any naturally occurring amino acid

<220>
<221>  misc_feature
<222>  (26)..(30)
<223>  Xaa can be any naturally occurring amino acid

<220>
<221>  misc_feature
<222>  (31)..(31)
<223>  hydrophobic amino acid

<220>
<221>  misc_feature
<222>  (32)..(32)
<223>  Xaa can be any naturally occurring amino acid

<400>  64

Xaa Xaa Xaa Cys Xaa Xaa Xaa Cys Xaa Xaa Xaa Xaa Xaa Arg Ser Asp
1               5                   10                  15

Ser Leu Gln Glu His Xaa Xaa Xaa His Xaa Xaa Xaa Xaa Xaa Xaa Xaa
            20                  25                  30

<210>  65
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  65

Cys Xaa Xaa Xaa Cys Xaa Xaa Xaa Xaa Xaa Arg Ser Gln Asp Leu Gln
1               5                   10                  15

Lys His Xaa Xaa Xaa His Xaa Xaa Xaa Xaa Xaa Xaa Xaa
            20                  25

<210>  66
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  66

Cys Xaa Xaa Xaa Cys Xaa Xaa Xaa Xaa Xaa Gln Ser Ser Asn Leu Gln
1               5                   10                  15

Asn His Xaa Xaa Xaa His Xaa Xaa Xaa Xaa Xaa Xaa Xaa
            20                  25

<210>  67
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  67

Cys Xaa Xaa Xaa Cys Xaa Xaa Xaa Xaa Xaa Arg Ser Ser His Leu Gln
1               5                   10                  15

Lys His Xaa Xaa Xaa His Xaa Xaa Xaa Xaa Xaa Xaa Xaa
            20                  25

<210>  68
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  68

Cys Xaa Xaa Xaa Cys Xaa Xaa Xaa Xaa Xaa Gln Ser Ser His Leu Gln
1               5                   10                  15


Lys His Xaa Xaa Xaa His Xaa Xaa Xaa Xaa Xaa Xaa Xaa
            20                  25


<210>  69
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  69

Cys Xaa Xaa Xaa Cys Xaa Xaa Xaa Xaa Xaa Asp Ser Ser His Leu Gln
1               5                   10                  15


Lys His Xaa Xaa Xaa His Xaa Xaa Xaa Xaa Xaa Xaa Xaa
            20                  25


<210>  70
<211>  29
<212>  PRT
<213>  artificial sequence

<220>
<223>  C2H2-zinc-finger-motive


<220>

<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  70

Cys Xaa Xaa Xaa Cys Xaa Xaa Xaa Xaa Xaa Glu Ser Ser His Leu Gln
1               5                   10                  15

Lys His Xaa Xaa Xaa His Xaa Xaa Xaa Xaa Xaa Xaa Xaa
            20                  25

<210>  71
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>

<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  71

Cys Xaa Xaa Xaa Cys Xaa Xaa Xaa Xaa Xaa Arg Ser Ser His Leu Gln
1               5                   10                  15


Asn His Xaa Xaa Xaa His Xaa Xaa Xaa Xaa Xaa Xaa Xaa
            20                  25


<210>  72
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>

<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  72

Cys Xaa Xaa Xaa Cys Xaa Xaa Xaa Xaa Xaa Arg Ser Ser His Leu Gln
1               5                   10                  15


Thr His Xaa Xaa Xaa His Xaa Xaa Xaa Xaa Xaa Xaa Xaa
            20                  25


<210>  73
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>

<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  73

Cys Xaa Xaa Xaa Cys Xaa Xaa Xaa Xaa Xaa Arg Ser Ser His Leu Gln
1               5                   10                  15


Glu His Xaa Xaa Xaa His Xaa Xaa Xaa Xaa Xaa Xaa Xaa
            20                  25


<210>  74
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>

<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  74

Cys Xaa Xaa Xaa Cys Xaa Xaa Xaa Xaa Xaa Arg Ser Gly Asn Leu Gln
1               5                   10                  15


Asn His Xaa Xaa Xaa His Xaa Xaa Xaa Xaa Xaa Xaa Xaa
            20                  25


<210>  75
<211>  29
<212>  PRT
<213>  artificial sequence

<220>
<223>  C2H2-zinc-finger-motive



<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>

<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  75

Cys Xaa Xaa Xaa Cys Xaa Xaa Xaa Xaa Xaa Glu Ser Gly Asn Leu Gln
1               5                   10                  15

Asn His Xaa Xaa Xaa His Xaa Xaa Xaa Xaa Xaa Xaa Xaa
            20                  25

<210>  76
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>

<221>  misc_feature
<222>  (28)..(28)
<223>  hydrophobic amino acid

<220>
<221>  misc_feature
<222>  (29)..(29)
<223>  Xaa can be any naturally occurring amino acid

<400>  76

Cys Xaa Xaa Xaa Cys Xaa Xaa Xaa Xaa Xaa Asp Ser Gly Asn Leu Gln
1               5                   10                  15

Asn His Xaa Xaa Xaa His Xaa Xaa Xaa Xaa Xaa Xaa Xaa
            20                  25

<210>  77
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>

<221>  misc_feature
<222>  (29)..(29)
<223>  Xaa can be any naturally occurring amino acid

<400>  77

Cys Xaa Xaa Xaa Cys Xaa Xaa Xaa Xaa Xaa Gln Ser Gly Asn Leu Gln
1               5                   10                  15


Lys His Xaa Xaa Xaa His Xaa Xaa Xaa Xaa Xaa Xaa Xaa
            20                  25


<210>  78
<211>  29
<212>  PRT
<213>  artificial sequence

<220>
<223>  C2H2-zinc-finger-motive



<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  78

Cys Xaa Xaa Xaa Cys Xaa Xaa Xaa Xaa Xaa Gln Ser Gly Asn Leu Gln
1               5                   10                  15

Thr His Xaa Xaa Xaa His Xaa Xaa Xaa Xaa Xaa Xaa Xaa
            20                  25

<210>  79
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  79

Cys Xaa Xaa Xaa Cys Xaa Xaa Xaa Xaa Xaa Gln Ser Gly Asn Leu Gln
1               5                   10                  15

Glu His Xaa Xaa Xaa His Xaa Xaa Xaa Xaa Xaa Xaa Xaa
            20                  25

<210>  80
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  80

Cys Xaa Xaa Xaa Cys Xaa Xaa Xaa Xaa Xaa Glu Ser Asp Ser Leu Gln
1               5                   10                  15

Thr His Xaa Xaa Xaa His Xaa Xaa Xaa Xaa Xaa Xaa Xaa
            20                  25

<210>  81

<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  81

Cys Xaa Xaa Xaa Cys Xaa Xaa Xaa Xaa Xaa Arg Ser Asp Ser Leu Gln
1               5                   10                  15


Thr His Xaa Xaa Xaa His Xaa Xaa Xaa Xaa Xaa Xaa Xaa
            20                  25


<210>  82
<211>  29
<212>  PRT
<213>  artificial sequence

<220>

<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  82

Cys Xaa Xaa Xaa Cys Xaa Xaa Xaa Xaa Xaa Gln Ser Asp Ser Leu Gln
1               5                   10                  15

Thr His Xaa Xaa Xaa His Xaa Xaa Xaa Xaa Xaa Xaa Xaa
            20                  25

<210>  83
<211>  29
<212>  PRT
<213>  artificial sequence

<220>
<223>  C2H2-zinc-finger-motive

<220>
<221>  misc_feature

<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  83

Cys Xaa Xaa Xaa Cys Xaa Xaa Xaa Xaa Xaa Asp Ser Asp Ser Leu Gln
1               5                   10                  15

Thr His Xaa Xaa Xaa His Xaa Xaa Xaa Xaa Xaa Xaa Xaa
            20                  25

<210>  84
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature

<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  84

Cys Xaa Xaa Xaa Cys Xaa Xaa Xaa Xaa Xaa Glu Ser Asp Ser Leu Gln
1               5                   10                  15

Lys His Xaa Xaa Xaa His Xaa Xaa Xaa Xaa Xaa Xaa Xaa
            20                  25

<210>  85
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature

<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  85

Cys Xaa Xaa Xaa Cys Xaa Xaa Xaa Xaa Xaa Glu Ser Asp Ser Leu Gln
1               5                   10                  15


Asn His Xaa Xaa Xaa His Xaa Xaa Xaa Xaa Xaa Xaa Xaa
            20                  25


<210>  86
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature

<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  86

Cys Xaa Xaa Xaa Cys Xaa Xaa Xaa Xaa Xaa Glu Ser Asp Ser Leu Gln
1               5                   10                  15


Glu His Xaa Xaa Xaa His Xaa Xaa Xaa Xaa Xaa Xaa Xaa
            20                  25


<210>  87
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature

<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  87

Cys Xaa Xaa Xaa Cys Xaa Xaa Xaa Xaa Xaa Asp Ser Gln Asp Leu Gln
1               5                   10                  15


Glu His Xaa Xaa Xaa His Xaa Xaa Xaa Xaa Xaa Xaa Xaa
            20                  25


<210>  88
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature

<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  88

Cys Xaa Xaa Xaa Cys Xaa Xaa Xaa Xaa Xaa Arg Ser Gln Asp Leu Gln
1               5                   10                  15

Glu His Xaa Xaa Xaa His Xaa Xaa Xaa Xaa Xaa Xaa Xaa
            20                  25

<210>  89
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature

<222>  (28)..(28)
<223>  hydrophobic amino acid

<220>
<221>  misc_feature
<222>  (29)..(29)
<223>  Xaa can be any naturally occurring amino acid

<400>  89

Cys Xaa Xaa Xaa Cys Xaa Xaa Xaa Xaa Xaa Gln Ser Gln Asp Leu Gln
1               5                   10                  15

Glu His Xaa Xaa Xaa His Xaa Xaa Xaa Xaa Xaa Xaa Xaa
            20                  25

<210>  90
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature

<222>  (29)..(29)
<223>  Xaa can be any naturally occurring amino acid

<400>  90

Cys Xaa Xaa Xaa Cys Xaa Xaa Xaa Xaa Xaa Glu Ser Gln Asp Leu Gln
1               5                   10                  15

Glu His Xaa Xaa Xaa His Xaa Xaa Xaa Xaa Xaa Xaa Xaa
            20                  25

<210>  91
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  91

Cys Xaa Xaa Xaa Cys Xaa Xaa Xaa Xaa Xaa Asp Ser Gln Asp Leu Gln
1               5                   10                  15

Lys His Xaa Xaa Xaa His Xaa Xaa Xaa Xaa Xaa Xaa Xaa
            20                  25

<210>  92
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  92

Cys Xaa Xaa Xaa Cys Xaa Xaa Xaa Xaa Xaa Asp Ser Gln Asp Leu Gln
1               5                   10                  15

Asn His Xaa Xaa Xaa His Xaa Xaa Xaa Xaa Xaa Xaa Xaa

20                    25

<210>  93
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  93

Cys Xaa Xaa Xaa Cys Xaa Xaa Xaa Xaa Xaa Asp Ser Gln Asp Leu Gln
1               5                   10                  15

Thr His Xaa Xaa Xaa His Xaa Xaa Xaa Xaa Xaa Xaa Xaa
            20                  25

<210>  94
<211>  29

<212>  PRT
<213>  artificial sequence

<220>
<223>  2H2-zinc-finger-motive



<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  94

Cys Xaa Xaa Xaa Cys Xaa Xaa Xaa Xaa Xaa Gln Ser Gln Asp Leu Gln
1               5                   10                  15


Lys His Xaa Xaa Xaa His Xaa Xaa Xaa Xaa Xaa Xaa Xaa
            20                  25


<210>  95
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  95

Cys Xaa Xaa Xaa Cys Xaa Xaa Xaa Xaa Xaa Glu Ser Gln Asp Leu Gln
1               5                   10                  15


Lys His Xaa Xaa Xaa His Xaa Xaa Xaa Xaa Xaa Xaa Xaa
            20                  25


<210>  96
<211>  29
<212>  PRT
<213>  artificial sequence

<220>
<223>  C2H2-zinc-finger-motive


<220>
<221>  misc_feature
<222>  (2)..(4)

<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  96

Cys Xaa Xaa Xaa Cys Xaa Xaa Xaa Xaa Xaa Arg Ser Gly Asn Leu Gln
1               5                   10                  15

Lys His Xaa Xaa Xaa His Xaa Xaa Xaa Xaa Xaa Xaa Xaa
            20                  25

<210>  97
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)

<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  97

Cys Xaa Xaa Xaa Cys Xaa Xaa Xaa Xaa Xaa Glu Ser Gly Asn Leu Gln
1               5                   10                  15


Lys His Xaa Xaa Xaa His Xaa Xaa Xaa Xaa Xaa Xaa Xaa
            20                  25


<210>  98
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)

<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  98

Cys Xaa Xaa Xaa Cys Xaa Xaa Xaa Xaa Xaa Asp Ser Gly Asn Leu Gln
1               5                   10                  15

Lys His Xaa Xaa Xaa His Xaa Xaa Xaa Xaa Xaa Xaa Xaa
            20                  25

<210>  99
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)

<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  99

Cys Xaa Xaa Xaa Cys Xaa Xaa Xaa Xaa Xaa Arg Ser Asp Ser Leu Gln
1               5                   10                  15


Lys His Xaa Xaa Xaa His Xaa Xaa Xaa Xaa Xaa Xaa Xaa
            20                  25


<210>  100
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)

<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  100

Cys Xaa Xaa Xaa Cys Xaa Xaa Xaa Xaa Xaa Gln Ser Asp Ser Leu Gln
1               5                   10                  15


Lys His Xaa Xaa Xaa His Xaa Xaa Xaa Xaa Xaa Xaa Xaa
            20                  25


<210>  101
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)

<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  101

Cys Xaa Xaa Xaa Cys Xaa Xaa Xaa Xaa Xaa Asp Ser Asp Ser Leu Gln
1               5                   10                  15


Lys His Xaa Xaa Xaa His Xaa Xaa Xaa Xaa Xaa Xaa Xaa
            20                  25


<210>  102
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)

<223>  hydrophobic amino acid

<220>
<221>  misc_feature
<222>  (29)..(29)
<223>  Xaa can be any naturally occurring amino acid

<400>  102

Cys Xaa Xaa Xaa Cys Xaa Xaa Xaa Xaa Xaa Gln Ser Ser His Leu Gln
1               5                   10                  15

Asn His Xaa Xaa Xaa His Xaa Xaa Xaa Xaa Xaa Xaa Xaa
            20                  25

<210>  103
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)

<223>  Xaa can be any naturally occurring amino acid

<400>  103

Cys Xaa Xaa Xaa Cys Xaa Xaa Xaa Xaa Xaa Gln Ser Gln Asp Leu Gln
1               5                   10                  15

Asn His Xaa Xaa Xaa His Xaa Xaa Xaa Xaa Xaa Xaa Xaa
            20                  25

<210>  104
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  104

Cys Xaa Xaa Xaa Cys Xaa Xaa Xaa Xaa Xaa Gln Ser Asp Ser Leu Gln

1                5                   10                  15

Asn His Xaa Xaa Xaa His Xaa Xaa Xaa Xaa Xaa Xaa Xaa
            20                  25

<210>  105
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  105

Cys Xaa Xaa Xaa Cys Xaa Xaa Xaa Xaa Xaa Glu Ser Gln Asp Leu Gln
1               5                   10                  15

Asn His Xaa Xaa Xaa His Xaa Xaa Xaa Xaa Xaa Xaa Xaa
            20                  25

<210>  106
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  106

Cys Xaa Xaa Xaa Cys Xaa Xaa Xaa Xaa Xaa Arg Ser Gln Asp Leu Gln
1               5                   10                  15


Asn His Xaa Xaa Xaa His Xaa Xaa Xaa Xaa Xaa Xaa Xaa
            20                  25


<210>  107
<211>  29
<212>  PRT

<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  107

Cys Xaa Xaa Xaa Cys Xaa Xaa Xaa Xaa Xaa Asp Ser Asp Ser Leu Gln
1               5                   10                  15


Asn His Xaa Xaa Xaa His Xaa Xaa Xaa Xaa Xaa Xaa Xaa
            20                  25


<210>  108
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  108

Cys Xaa Xaa Xaa Cys Xaa Xaa Xaa Xaa Xaa Asp Ser Ser His Leu Gln
1               5                   10                  15

Asn His Xaa Xaa Xaa His Xaa Xaa Xaa Xaa Xaa Xaa Xaa
            20                  25

<210>  109
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  109

Cys Xaa Xaa Xaa Cys Xaa Xaa Xaa Xaa Xaa Glu Ser Ser His Leu Gln
1               5                   10                  15


Asn His Xaa Xaa Xaa His Xaa Xaa Xaa Xaa Xaa Xaa Xaa
            20                  25


<210>  110
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  110

Cys Xaa Xaa Xaa Cys Xaa Xaa Xaa Xaa Xaa Arg Ser Asp Ser Leu Gln
1               5                   10                  15

Asn His Xaa Xaa Xaa His Xaa Xaa Xaa Xaa Xaa Xaa Xaa
            20                  25

<210>  111
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  111

Cys Xaa Xaa Xaa Cys Xaa Xaa Xaa Xaa Xaa Glu Ser Gly Asn Leu Gln
1               5                   10                  15


Thr His Xaa Xaa Xaa His Xaa Xaa Xaa Xaa Xaa Xaa Xaa
            20                  25


<210>  112
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  112

Cys Xaa Xaa Xaa Cys Xaa Xaa Xaa Xaa Xaa Glu Ser Ser His Leu Gln
1               5                   10                  15

Thr His Xaa Xaa Xaa His Xaa Xaa Xaa Xaa Xaa Xaa Xaa
            20                  25

<210>  113
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  113

Cys Xaa Xaa Xaa Cys Xaa Xaa Xaa Xaa Xaa Glu Ser Gln Asp Leu Gln
1               5                   10                  15

Thr His Xaa Xaa Xaa His Xaa Xaa Xaa Xaa Xaa Xaa Xaa
            20                  25

<210>  114
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  114

Cys Xaa Xaa Xaa Cys Xaa Xaa Xaa Xaa Xaa Gln Ser Gln Asp Leu Gln
1               5                   10                  15


Thr His Xaa Xaa Xaa His Xaa Xaa Xaa Xaa Xaa Xaa Xaa
            20                  25


<210>  115
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  115

Cys Xaa Xaa Xaa Cys Xaa Xaa Xaa Xaa Xaa Arg Ser Gln Asp Leu Gln
1               5                   10                  15

Thr His Xaa Xaa Xaa His Xaa Xaa Xaa Xaa Xaa Xaa Xaa
            20                  25

<210>  116
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  116

Cys Xaa Xaa Xaa Cys Xaa Xaa Xaa Xaa Xaa Asp Ser Gly Asn Leu Gln
1               5                   10                  15

Thr His Xaa Xaa Xaa His Xaa Xaa Xaa Xaa Xaa Xaa Xaa
            20                  25

<210>  117
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  117

Cys Xaa Xaa Xaa Cys Xaa Xaa Xaa Xaa Xaa Asp Ser Ser His Leu Gln
1               5                   10                  15

Thr His Xaa Xaa Xaa His Xaa Xaa Xaa Xaa Xaa Xaa Xaa
            20                  25

<210>  118
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  118

Cys Xaa Xaa Xaa Cys Xaa Xaa Xaa Xaa Xaa Arg Ser Gly Asn Leu Gln
1               5                   10                  15

Thr His Xaa Xaa Xaa His Xaa Xaa Xaa Xaa Xaa Xaa Xaa
            20                  25

<210>  119
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  119

Cys Xaa Xaa Xaa Cys Xaa Xaa Xaa Xaa Xaa Gln Ser Ser His Leu Gln
1               5                   10                  15


Thr His Xaa Xaa Xaa His Xaa Xaa Xaa Xaa Xaa Xaa Xaa
            20                  25


<210>  120
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  120

Cys Xaa Xaa Xaa Cys Xaa Xaa Xaa Xaa Xaa Asp Ser Gly Asn Leu Gln
1               5                   10                  15

Glu His Xaa Xaa Xaa His Xaa Xaa Xaa Xaa Xaa Xaa Xaa
            20                  25

<210>  121
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  121

Cys Xaa Xaa Xaa Cys Xaa Xaa Xaa Xaa Xaa Asp Ser Asp Ser Leu Gln
1               5                   10                  15


Glu His Xaa Xaa Xaa His Xaa Xaa Xaa Xaa Xaa Xaa Xaa
            20                  25


<210>  122
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  122

Cys Xaa Xaa Xaa Cys Xaa Xaa Xaa Xaa Xaa Asp Ser Ser His Leu Gln
1               5                   10                  15


Glu His Xaa Xaa Xaa His Xaa Xaa Xaa Xaa Xaa Xaa Xaa
            20                  25


<210>  123
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  123

Cys Xaa Xaa Xaa Cys Xaa Xaa Xaa Xaa Xaa Gln Ser Ser His Leu Gln
1               5                   10                  15


Glu His Xaa Xaa Xaa His Xaa Xaa Xaa Xaa Xaa Xaa Xaa
            20                  25


<210>  124
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  124

Cys Xaa Xaa Xaa Cys Xaa Xaa Xaa Xaa Xaa Glu Ser Ser His Leu Gln
1               5                   10                  15


Glu His Xaa Xaa Xaa His Xaa Xaa Xaa Xaa Xaa Xaa Xaa
            20                  25


<210>  125
<211>  29
<212>  PRT
<213>  artificial sequence

<220>
<223>  C2H2-zinc-finger-motive



<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  125

Cys Xaa Xaa Xaa Cys Xaa Xaa Xaa Xaa Xaa Arg Ser Gly Asn Leu Gln
1               5                   10                  15


Glu His Xaa Xaa Xaa His Xaa Xaa Xaa Xaa Xaa Xaa Xaa
            20                  25


<210>  126
<211>  29
<212>  PRT
<213>  artificial sequence

<220>
<223>  C2H2-zinc-finger-motive


<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  126

Cys Xaa Xaa Xaa Cys Xaa Xaa Xaa Xaa Xaa Glu Ser Gly Asn Leu Gln
1               5                   10                  15

Glu His Xaa Xaa Xaa His Xaa Xaa Xaa Xaa Xaa Xaa Xaa
            20                  25

<210>  127
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  127

Cys Xaa Xaa Xaa Cys Xaa Xaa Xaa Xaa Xaa Gln Ser Asp Ser Leu Gln
1               5                   10                  15

Glu His Xaa Xaa Xaa His Xaa Xaa Xaa Xaa Xaa Xaa Xaa
            20                  25

<210>  128
<211>  29
<212>  PRT
<213>  artificial sequence

<220>
<223>  C2H2-zinc-finger-motive

<220>
<221>  misc_feature
<222>  (2)..(4)
<223>  Xaa can be any naturally occurring amino acid

<220>
<221>  misc_feature
<222>  (6)..(8)
<223>  Xaa can be any naturally occurring amino acid

<220>
<221>  misc_feature
<222>  (9)..(9)
<223>  hydrophobic amino acid

<220>
<221>  misc_feature
<222>  (10)..(10)
<223>  Xaa can be any naturally occurring amino acid

<220>
<221>  misc_feature
<222>  (19)..(21)
<223>  Xaa can be any naturally occurring amino acid

<220>
<221>  misc_feature
<222>  (23)..(27)
<223>  Xaa can be any naturally occurring amino acid

<220>
<221>  misc_feature
<222>  (28)..(28)
<223>  hydrophobic amino acid

<220>
<221>  misc_feature
<222>  (29)..(29)
<223>  Xaa can be any naturally occurring amino acid

<400>  128

Cys Xaa Xaa Xaa Cys Xaa Xaa Xaa Xaa Xaa Arg Ser Asp Ser Leu Gln
1               5                   10                  15

Glu His Xaa Xaa Xaa His Xaa Xaa Xaa Xaa Xaa Xaa Xaa
            20                  25

### Anhang

### Sequenzprotokoll 1

Bibliothek 1; das Carboxylende trägt eine Thioester; als Funktionalisierung ist hier exemplarisch die Fluorophore Rhodamin-B genannt (siehe Aminoterminus). X steht für eine beliebige Aminosäure; Z steht für eine hydrophobe Aminosäure; die übrigen Aminosäuren sind im international gültigen "One Letter Code" geschrieben.

-11234 56
dC2H2D-1: Rhod-B-XZXCXXXCXXXZXRSQDLQKHXXXHXXXXXZX    R-QD-K an G-C-G

-1 23   6
dC2H2D-2: Rhod-B-XZXCXXXCXXXZXQSSNLQNHXXXHXXXXXZX    Q-SN-N an A-A-A

-1 23   6
dC2H2D10: Rhod-B-XZXCXXXCXXXZXRSGNLQNHXXXHXXXXXZX    R-GN-N an A-A-G

-1 23   6
dC2H2D-3: Rhod-B-XZXCXXXCXXXZXRSSHLQKHXXXHXXXXXZX    R-SH-K an G-G-G

-1 23   6
dC2H2D-4: Rhod-B-XZXCXXXCXXXZXQSSHLQKHXXXHXXXXXZX    Q-SH-K an G-G-A

-1 23   6
dC2H2D-5: Rhod-B-XZXCXXXCXXXZXDSSHLQKHXXXHXXXXXZX    D-SH-K an G-G-C

-1 23   6
dC2H2D-6: Rhod-B-XZXCXXXCXXXZXESSHLQKHXXXHXXXXXZX    E-SH-K an G-G-T

-1 23   6
dC2H2D-7: Rhod-B-XZXCXXXCXXXZXRSSHLQNHXXXHXXXXXZX    R-SH-N an A-G-G

-1 23   6
dC2H2D-8: Rhod-B-XZXCXXXCXXXZXRSSHLQTHXXXHXXXXXZX    R-SH-T an T-G-G

-1 23 6
dC2H2D-9: Rhod-B-XZXCXXXCXXXZXRSSHLQEHXXXHXXXXXZX R-SH-E an C-G-G

-1 23 6
dC2H2D11: Rhod-B-XZXCXXXCXXXZXESGNLQNHXXXHXXXXXZX E-GN-T an A-A-T

-1 23 6
dC2H2D12: Rhod-B-XZXCXXXCXXXZXDSGNLQNHXXXHXXXXXZX D-GN-T an A-A-C

-1 23 6
dC2H2D13: Rhod-B-XZXCXXXCXXXZXQSGNLQKHXXXHXXXXXZX Q-GN-K an G-A-A

-1 23 6
dC2H2D14: Rhod-B-XZXCXXXCXXXZXQSGNLQTHXXXHXXXXXZX Q-GN-T an T-A-A

-1 23 6
dC2H2D15: Rhod-B-XZXCXXXCXXXZXQSGNLQEHXXXHXXXXXZX Q-GN-E an C-A-A

-1 23 6
dC2H2D16: Rhod-B-XZXCXXXCXXXZXESDSLQTHXXXHXXXXXZX E-DS-T an T-T-T

-1 23 6
dC2H2D17: Rhod-B-XZXCXXXCXXXZXRSDSLQTHXXXHXXXXXZX R-DS-T an T-T-G

-1 23 6
dC2H2D18: Rhod-B-XZXCXXXCXXXZXQSDSLQTHXXXHXXXXXZX Q-DS-T an T-T-A

-1 23 6
dC2H2D19: Rhod-B-XZXCXXXCXXXZXDSDSLQTHXXXHXXXXXZX D-DS-T an T-T-C

-1 23 6
dC2H2D20: Rhod-B-XZXCXXXCXXXZXESDSLQKHXXXHXXXXXZX E-DS-K an G-T-T

-1 23 6
dC2H2D21: Rhod-B-XZXCXXXCXXXZXESDSLQNHXXXHXXXXXZX E-DS-T an A-T-T

-1 23 6
dC2H2D22: Rhod-B-XZXCXXXCXXXZXESDSLQEHXXXHXXXXXZX E-DS-E an C-T-T

-1 23 6
dC2H2D23: Rhod-B-XZXCXXXCXXXZXDSQDLQEHXXXHXXXXXZX D-QD-E an C-C-C

-1 23 6
dC2H2D24: Rhod-B-XZXCXXXCXXXZXRSQDLQEHXXXHXXXXXZX R-QD-E an C-C-G

-1 23 6
dC2H2D25: Rhod-B-XZXCXXXCXXXZXQSQDLQEHXXXHXXXXXZX Q-QD-E an C-C-A

-1 23 6
dC2H2D26: Rhod-B-XZXCXXXCXXXZXESQDLQEHXXXHXXXXXZX E-QD-E an C-C-T

-1 23 6
dC2H2D27: Rhod-B-XZXCXXXCXXXZXDSQDLQKHXXXHXXXXXZX D-QD-K an G-C-C

-1 23 6
dC2H2D28: Rhod-B-XZXCXXXCXXXZXDSQDLQNHXXXHXXXXXZX D-QD-T an A-C-C

-1 23 6
dC2H2D29: Rhod-B-XZXCXXXCXXXZXDSQDLQTHXXXHXXXXXZX D-QD-T an T-C-C

-1 23 6
dC2H2D30: Rhod-B-XZXCXXXCXXXZXQSQDLQKHXXXHXXXXXZX Q-QD-K an G-C-A

-1 23 6
dC2H2D31: Rhod-B-XZXCXXXCXXXZXESQDLQKHXXXHXXXXXZX E-QD-K an G-C-T

-1 23 6
dC2H2D32: Rhod-B-XZXCXXXCXXXZXRSGNLQKHXXXHXXXXXZX R-GN-K an G-A-G

-1 23 6
dC2H2D33: Rhod-B-XZXCXXXCXXXZXESGNLQKHXXXHXXXXXZX E-GN-K an G-A-T

-1 23 6
dC2H2D34: Rhod-B-XZXCXXXCXXXZXDSGNLQKHXXXHXXXXXZX D-GN-K an G-A-C

-1 23 6
dC2H2D35: Rhod-B-XZXCXXXCXXXZXRSDSLQKHXXXHXXXXXZX R-DS-K an G-T-G

-1 23 6
dC2H2D36: Rhod-B-XZXCXXXCXXXZXQSDSLQKHXXXHXXXXXZX Q-DS-K an G-T-A

-1 23 6
dC2H2D37: Rhod-B-XZXCXXXCXXXZXDSDSLQKHXXXHXXXXXZX D-DS-K an G-T-C

-1 23 6
dC2H2D38: Rhod-B-XZXCXXXCXXXZXQSSHLQNHXXXHXXXXXZX Q-SH-T an A-G-A

-1 23 6
dC2H2D39: Rhod-B-XZXCXXXCXXXZXQSQDLQNHXXXHXXXXXZX Q-QD-T an A-C-A

-1 23 6
dC2H2D40: Rhod-B-XZXCXXXCXXXZXQSDSLQNHXXXHXXXXXZX Q-DS-T an A-T-A

-1 23 6
dC2H2D41: Rhod-B-XZXCXXXCXXXZXESQDLQNHXXXHXXXXXZX E-QD-T an A-C-T

-1 23 6
dC2H2D42: Rhod-B-XZXCXXXCXXXZXRSQDLQNHXXXHXXXXXZX R-QD-T an A-C-G

-1 23 6
dC2H2D43: Rhod-B-XZXCXXXCXXXZXDSDSLQNHXXXHXXXXXZX D-DS-T an A-T-C

-1 23 6
dC2H2D44: Rhod-B-XZXCXXXCXXXZXDSSHLQNHXXXHXXXXXZX D-SH-T an A-G-C

-1 23 6
dC2H2D45: Rhod-B-XZXCXXXCXXXZXESSHLQNHXXXHXXXXXZX E-SH-T an A-G-T

-1 23 6
dC2H2D46: Rhod-B-XZXCXXXCXXXZXRSDSLQNHXXXHXXXXXZX R-DS-T an A-T-G

-1 23 6
dC2H2D47: Rhod-B-XZXCXXXCXXXZXESGNLQTHXXXHXXXXXZX E-GN-T an T-A-T

-1 23 6
dC2H2D48: Rhod-B-XZXCXXXCXXXZXESSHLQTHXXXHXXXXXZX E-SH-T an T-G-T

-1 23 6
dC2H2D49: Rhod-B-XZXCXXXCXXXZXESQDLQTHXXXHXXXXXZX E-QD-T an T-C-T

-1 23 6
dC2H2D50: Rhod-B-XZXCXXXCXXXZXQSQDLQTHXXXHXXXXXZX Q-QD-T an T-C-A

-1 23 6
dC2H2D51: Rhod-B-XZXCXXXCXXXZXRSQDLQTHXXXHXXXXXZX R-QD-T an T-C-G

-1 23 6
dC2H2D52: Rhod-B-XZXCXXXCXXXZXDSGNLQTHXXXHXXXXXZX D-GN-T an T-A-C

-1 23 6
dC2H2D53: Rhod-B-XZXCXXXCXXXZXDSSHLQTHXXXHXXXXXZX D-SH-T an T-G-C

-1 23 6
dC2H2D54: Rhod-B-XZXCXXXCXXXZXRSGNLQTHXXXHXXXXXZX R-GN-T an T-A-G

-1 23 6
dC2H2D55: Rhod-B-XZXCXXXCXXXZXQSSHLQTHXXXHXXXXXZX Q-SH-T an T-G-A

-1 23 6
dC2H2D56: Rhod-B-XZXCXXXCXXXZXDSGNLQEHXXXHXXXXXZX D-GN-E an C-A-C

-1 23 6
dC2H2D57: Rhod-B-XZXCXXXCXXXZXDSDSLQEHXXXHXXXXXZX D-DS-E an C-T-C

-1 23 6
dC2H2D58: Rhod-B-XZXCXXXCXXXZXDSSHLQEHXXXHXXXXXZX D-SH-E an C-G-C

-1 23 6
dC2H2D59: Rhod-B-XZXCXXXCXXXZXQSSHLQEHXXXHXXXXXZX Q-SH-E an C-G-A

-1 23 6
dC2H2D60: Rhod-B-XZXCXXXCXXXZXESSHLQEHXXXHXXXXXZX E-SH-E an C-G-T

-1 23 6
dC2H2D61: Rhod-B-XZXCXXXCXXXZXRSGNLQEHXXXHXXXXXZX R-GN-E an C-A-G

-1 23 6
dC2H2D62: Rhod-B-XZXCXXXCXXXZXESGNLQEHXXXHXXXXXZX E-GN-E an C-A-T

-1 23 6
dC2H2D63: Rhod-B-XZXCXXXCXXXZXQSDSLQEHXXXHXXXXXZX Q-DS-E an C-T-A

-1 23 6
dC2H2D64: Rhod-B-XZXCXXXCXXXZXRSDSLQEHXXXHXXXXXZX R-DS-E an C-T-G

Die Aminosäure Nummer 6 korrespondiert mit der 5'-Base des Nukleinsäuretripletts.

### Sequenzprotokoll 2

Bibliotheken 2 und 3; die Aminosäuren von Bibliothek 2 tragen am Carboxyterminus (hier: X) einen aktivierten Thioester und das aminoterminale Cysteine ist zusätzlich über THZ geschützt. Die Peptide von Bibliothek 3 sind unmodifiziert (kein THZ, kein Thioester).

-1123456
dC2H2-1: CXXXCXXXZXRSQDLQKHXXXHXXXXXZX   R-QD-K an G-C-G

-1 23  6
dC2H2-2: CXXXCXXXZXQSSNLQNHXXXHXXXXXZX   Q-SN-N an A-A-A

-1 23  6
dC2H210: CXXXCXXXZXRSGNLQNHXXXHXXXXXZX   R-GN-N an A-A-G

-1 23  6
dC2H2-3: CXXXCXXXZXRSSHLQKHXXXHXXXXXZX   R-SH-K an G-G-G

-1 23  6
dC2H2-4: CXXXCXXXZXQSSHLQKHXXXHXXXXXZX   Q-SH-K an G-G-A

-1 23  6
dC2H2-5: CXXXCXXXZXDSSHLQKHXXXHXXXXXZX   D-SH-K an G-G-C

-1 23  6
dC2H2-6: CXXXCXXXZXESSHLQKHXXXHXXXXXZX   E-SH-K an G-G-T

-1 23  6
dC2H2-7: CXXXCXXXZXRSSHLQNHXXXHXXXXXZX   R-SH-N an A-G-G

-1 23  6
dC2H2-8: CXXXCXXXZXRSSHLQTHXXXHXXXXXZX   R-SH-T an T-G-G

-1 23  6
dC2H2-9: CXXXCXXXZXRSSHLQEHXXXHXXXXXZX   R-SH-E an C-G-G

-1 23 6
dC2H211: CXXXCXXXZXESGNLQNHXXXHXXXXXZX E-GN-N an A-A-T

-1 23 6
dC2H212: CXXXCXXXZXDSGNLQNHXXXHXXXXXZX D-GN-N an A-A-C

-1 23 6
dC2H213: CXXXCXXXZXQSGNLQKHXXXHXXXXXZX Q-GN-K an G-A-A

-1 23 6
dC2H214: CXXXCXXXZXQSGNLQTHXXXHXXXXXZX Q-GN-T an T-A-A

-1 23 6
dC2H215: CXXXCXXXZXQSGNLQEHXXXHXXXXXZX Q-GN-E an C-A-A

-1 23 6
dC2H216: CXXXCXXXZXESDSLQTHXXXHXXXXXZX E-DS-T an T-T-T

-1 23 6
dC2H217: CXXXCXXXZXRSDSLQTHXXXHXXXXXZX R-DS-T an T-T-G

-1 23 6
dC2H218: CXXXCXXXZXQSDSLQTHXXXHXXXXXZX Q-DS-T an T-T-A

-1 23 6
dC2H219: CXXXCXXXZXDSDSLQTHXXXHXXXXXZX D-DS-T an T-T-C

-1 23 6
dC2H220: CXXXCXXXZXESDSLQKHXXXHXXXXXZX E-DS-K an G-T-T

-1 23 6
dC2H221: CXXXCXXXZXESDSLQNHXXXHXXXXXZX E-DS-N an A-T-T

-1 23 6
dC2H222: CXXXCXXXZXESDSLQEHXXXHXXXXXZX E-DS-E an C-T-T

-1 23 6
dC2H223: CXXXCXXXZXDSQDLQEHXXXHXXXXXZX D-QD-E an C-C-C

-1 23 6
dC2H224: CXXXCXXXZXRSQDLQEHXXXHXXXXXZX R-QD-E an C-C-G

-1 23 6
dC2H225: CXXXCXXXZXQSQDLQEHXXXHXXXXXZX Q-QD-E an C-C-A

-1 23 6
dC2H226: CXXXCXXXZXESQDLQEHXXXHXXXXXZX E-QD-E an C-C-T

-1 23 6
dC2H227: CXXXCXXXZXDSQDLQKHXXXHXXXXXZX D-QD-K an G-C-C

-1 23 6
dC2H228: CXXXCXXXZXDSQDLQNHXXXHXXXXXZX D-QD-N an A-C-C

-1 23 6
dC2H229: CXXXCXXXZXDSQDLQTHXXXHXXXXXZX D-QD-T an T-C-C

-1 23 6
dC2H230: CXXXCXXXZXQSQDLQKHXXXHXXXXXZX Q-QD-K an G-C-A

-1 23 6
dC2H231: CXXXCXXXZXESQDLQKHXXXHXXXXXZX E-QD-K an G-C-T

-1 23 6
dC2H232: CXXXCXXXZXRSGNLQKHXXXHXXXXXZX R-GN-K an G-A-G

-1 23 6
dC2H233: CXXXCXXXZXESGNLQKHXXXHXXXXXZX E-GN-K an G-A-T

-1 23 6
dC2H234: CXXXCXXXZXDSGNLQKHXXXHXXXXXZX D-GN-K an G-A-C

-1 23 6
dC2H235: CXXXCXXXZXRSDSLQKHXXXHXXXXXZX R-DS-K an G-T-G

-1 23 6
dC2H236: CXXXCXXXZXQSDSLQKHXXXHXXXXXZX Q-DS-K an G-T-A

-1 23 6
dC2H237: CXXXCXXXZXDSDSLQKHXXXHXXXXXZX D-DS-K an G-T-C

-1 23 6
dC2H238: CXXXCXXXZXQSSHLQNHXXXHXXXXXZX Q-SH-N an A-G-A

-1 23 6
dC2H239: CXXXCXXXZXQSQDLQNHXXXHXXXXXZX Q-QD-N an A-C-A

-1 23 6
dC2H240: CXXXCXXXZXQSDSLQNHXXXHXXXXXZX Q-DS-N an A-T-A

-1 23 6
dC2H241: CXXXCXXXZXESQDLQNHXXXHXXXXXZX E-QD-N an A-C-T

-1 23 6
dC2H242: CXXXCXXXZXRSQDLQNHXXXHXXXXXZX R-QD-N an A-C-G

-1 23 6
dC2H243: CXXXCXXXZXDSDSLQNHXXXHXXXXXZX D-DS-N an A-T-C

-1 23 6
dC2H244: CXXXCXXXZXDSSHLQNHXXXHXXXXXZX D-SH-N an A-G-C

-1 23 6
dC2H245: CXXXCXXXZXESSHLQNHXXXHXXXXXZX E-SH-N an A-G-T

-1 23 6
dC2H246: CXXXCXXXZXRSDSLQNHXXXHXXXXXZX R-DS-N an A-T-G

-1 23 6
dC2H247: CXXXCXXXZXESGNLQTHXXXHXXXXXZX E-GN-T an T-A-T

-1 23 6
dC2H248: CXXXCXXXZXESSHLQTHXXXHXXXXXZX E-SH-T an T-G-T

-1 23 6
dC2H249: CXXXCXXXZXESQDLQTHXXXHXXXXXZX E-QD-T an T-C-T

-1 23 6
dC2H250: CXXXCXXXZXQSQDLQTHXXXHXXXXXZX Q-QD-T an T-C-A

-1 23 6
dC2H251: CXXXCXXXZXRSQDLQTHXXXHXXXXXZX R-QD-T an T-C-G

-1 23 6
dC2H252: CXXXCXXXZXDSGNLQTHXXXHXXXXXZX D-GN-T an T-A-C

-1 23 6
dC2H253: CXXXCXXXZXDSSHLQTHXXXHXXXXXZX D-SH-T an T-G-C

-1 23 6
dC2H254: CXXXCXXXZXRSGNLQTHXXXHXXXXXZX R-GN-T an T-A-G

-1 23 6
dC2H255: CXXXCXXXZXQSSHLQTHXXXHXXXXXZX Q-SH-T an T-G-A

-1 23 6
dC2H256: CXXXCXXXZXDSGNLQEHXXXHXXXXXZX D-GN-E an C-A-C

-1 23  6
dC2H257: CXXXCXXXZXDSDSLQEHXXXHXXXXXZX   D-DS-E an C-T-C

-1 23  6
dC2H258: CXXXCXXXZXDSSHLQEHXXXHXXXXXZX   D-SH-E an C-G-C

-1 23  6
dC2H259: CXXXCXXXZXQSSHLQEHXXXHXXXXXZX   Q-SH-E an C-G-A

-1 23  6
dC2H260: CXXXCXXXZXESSHLQEHXXXHXXXXXZX   E-SH-E an C-G-T

-1 23  6
dC2H261: CXXXCXXXZXRSGNLQEHXXXHXXXXXZX   R-GN-E an C-A-G

-1 23  6
dC2H262: CXXXCXXXZXESGNLQEHXXXHXXXXXZX   E-GN-E an C-A-T

-1 23  6
dC2H263: CXXXCXXXZXQSDSLQEHXXXHXXXXXZX   Q-DS-E an C-T-A

-1 23  6
dC2H264: CXXXCXXXZXRSDSLQEHXXXHXXXXXZX   R-DS-E an C-T-G

Die Aminosäure Nummer 6 korrespondiert mit der 5'-Base des Nukleinsäuretripletts.

## Patentansprüche

1. Polyzinkfingermolekülbinder, bestehend aus **chemisch synthetisierten** Peptiden mit C2H2-Zinkfingermotiv, **dadurch gekennzeichnet,**
**dass** diese Peptide in Kombination und in Form von Multimeren an jede beliebige Doppelstrang-DNA binden und dass dieser Doppelstrang-DNA-Abschnitt **frei wählbar** ist.

2. Polyzinkfingermolekülbinder, synthetisiert durch Ligation von Bibliothek I nach Anspruch 5 bis 6, mit Bibliothek II nach Anspruch 7 und/oder 8 sowie mit Bibliothek III nach Anspruch 9.

3. Polyzinkfingermolekülbinder nach Anspruch 2, wobei die Ligation folgende Schritte umfasst:
a) Ein Molekül aus Bibliothek III wird mit einem Molekül aus Bibliothek II über das freie Cystein mit dem Thioester ligiert;
b) Entschützen des aus Bibliothek II stammenden Cysteins dieses in Schritt a) entstandenen Doppelmoleküls;
c) Ligation dieses modifizierten Doppelmoleküls mit einem weiteren Molekül aus Bibliothek II über das nun freie Cystein mit dem Thioester des dritten Moleküls zu einem Polypeptid mit drei Zinkfingerpeptiden;
d) Entschützen des am Aminoterminus befindliche Cystein dieses in Schritt c entstandenen Moleküls:
e) Hinzufügen eines weiteren Moleküls aus Bibliothek II durch Ligation wie in Schritt a) bis d) beschrieben;
f) Wiederholen von Schritt c) bis die gewünschte Anzahl von C2H2-Zinkfingerpeptiden erreicht ist.
g) Finale Ligation mit einem Molekül aus Bibliothek I oder Bibliothek II, wobei im letzteren Fall die Funktionalisierung nachträglich über das aminoterminale zu entschützende Cystein dieses letzten Bibliothek-II-Peptids erfolgen kann.

4. Polyzinkfingermolekülbinder, synthetisiert durch Ligation von Bibliothek II nach Anspruch 7 und/oder 8 mit Bibliothek III nach Anspruch 9.

5. Bibliothek I von synthetischen Peptiden mit C2H2-Zinkfingermotiv, wobei die Peptide,
(i) aminoterminal eine Funktionalisierung tragen können;
(ii) am Carboxyterminus die ersten zwei Aminosäuren des Folgepeptids der Ligation tragen.
iii) wobei die Aminosäure des absoluten Carboxyterminus einen Thioester enthält.

6. Bibliothek I nach Anspruch 5, wobei die synthetischen Peptide carboxyterminal einen Spacer tragen können.

7. Bibliothek II von synthetischen Peptiden mit C2H2-Zinkfingermotiv, wobei die Peptide
(i) aminoterminal mit einem Cystein beginnen, welches durch
eine Schutzgruppe inaktiviert ist;
(ii)am Carboxyterminus die ersten zwei Aminosäuren des
Folgepeptids der Ligation tragen
iii) wobei die Aminosäure des absoluten Carboxyterminus einen
Thioester enthält.

8. Bibliothek II nach Anspruch 8, wobei die synthetischen Peptide carboxyterminal einen Spacer tragen können.

9. Bibliothek III von synthetischen Peptiden mit C2H2-Zinkfingermotiv, wobei die Peptide aminoterminal mit einem Cystein beginnen, wobei dieses ungeschützt ist.

10. Bibliothek I nach Sequenzprotokoll 1.

11. Bibliothek II nach Sequenzprotokoll 2.

12. Bibliothek III nach Sequenzprotokoll 2.

13. Verwendung von Polyzinkfingermolekülbindern nach Anspruch 1 bis 4 in der molekularen Analytik.

14. Verwendung von Polyzinkfingermolekülbindern nach Anspruch 1 bis 4 als Sonden in der DNA-Analytik.

15. Biochip, auf dem Polyzinkfingermolekülbindern nach Anspruch 1 bis 4 immobilisiert ist.

16. Verfahren zur Herstellung von Polyzinkfingermolekülbindern nach einem oder mehreren der Ansprüche 1 bis 4, folgende Schritte umfassend:
a) Ein Molekül aus Bibliothek III wird mit einem Molekül aus Bibliothek II über das freie Cystein mit dem Thioester ligiert;
b) Entschützen des aus Bibliothek II stammenden Cysteins dieses in Schritt a) entstandenen Doppelmoleküls;
c) Ligation dieses modifizierten Doppelmoleküls mit einem weiteren Molekül aus Bibliothek II über das nun freie Cystein mit dem Thioester des dritten Moleküls zu einem Polypeptid mit drei Zinkfingerpeptiden;
d) Entschützen des am Aminoterminus befindliche Cystein dieses in Schritt c entstandenen Moleküls:
e) Hinzufügen eines weiteren Moleküls aus Bibliothek II durch Ligation wie in Schritt a) bis d) beschrieben;
f) Wiederholen von Schritt c) bis die gewünschte Anzahl von C2H2-Zinkfingerpeptiden erreicht ist.
g) Finale Ligation mit einem Molekül aus Bibliothek I oder Bibliothek II, wobei im letzteren Fall die Funktionalisierung nachträglich über das aminoterminale zu entschützende Cystein dieses letzten Bibliothek-II-Peptids erfolgen kann Vorrichtung zum...
